(19) Europäisches Patentamt
European Patent Office
Office européen des brevets

(11) EP 3 949 797 A1

(12) **EUROPEAN PATENT APPLICATION**
published in accordance with Art. 153(4) EPC

(43) Date of publication:
**09.02.2022 Bulletin 2022/06**

(21) Application number: **20782468.1**

(22) Date of filing: **06.04.2020**

(51) International Patent Classification (IPC):
***A45D 19/02*** (2006.01) ***A45D 34/04*** (2006.01)
***A61Q 5/00*** (2006.01) ***A61Q 5/06*** (2006.01)
***A61K 8/02*** (2006.01) ***A61K 8/891*** (2006.01)
***A61K 8/898*** (2006.01)

(52) Cooperative Patent Classification (CPC):
**A45D 19/02; A45D 34/04; A61K 8/02; A61K 8/891; A61K 8/898; A61Q 5/00; A61Q 5/06**

(86) International application number:
**PCT/JP2020/015486**

(87) International publication number:
**WO 2020/204207 (08.10.2020 Gazette 2020/41)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **04.04.2019 JP 2019072124**
**25.12.2019 JP 2019234973**

(71) Applicant: **Kao Corporation**
**Chuo-ku**
**Tokyo 103-8210 (JP)**

(72) Inventors:
- **SAITO, Yoichi**
**Tokyo 131-8501 (JP)**
- **NAKAOKA, Shiho**
**Tokyo 131-8501 (JP)**
- **ASANO, Tetsuya**
**Tokyo 131-8501 (JP)**

(74) Representative: **Hoffmann Eitle**
**Patent- und Rechtsanwälte PartmbB**
**Arabellastraße 30**
**81925 München (DE)**

(54) **COSMETIC PRODUCT FOR HAIR**

(57) The present invention relates to a cosmetic for hair 100 including a container 1 having a liquid hair cosmetic preparation L accommodated in the inside thereof; and an applicator 2 provided with not only an application body 4 at one end of an application shaft 3 but also a container lid part 5 at the other end of the application shaft 3, wherein the hair cosmetic preparation L has a viscosity at 25°C of 5,000 mPa·s or less; and the application body 4 is provided with an absorptive member having a density of 15 kg/$m^3$ or more and 95 kg/$m^3$ or less, and the absorptive member covers the entirety of the one end of the application shaft 3.

[Fig. 2]

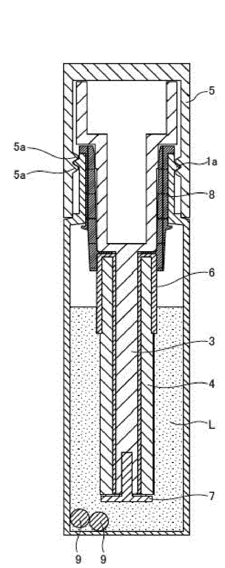


EP 3 949 797 A1

## Description

Field of the Invention

The present invention relates to a cosmetic for hair.

Background of the Invention

A hair dye is classified into a permanent hair dye in which an oxidative dye is used for color development to produce a color in hair (e.g., a hair coloring), a semi-permanent hair dye in which a direct dye is subjected to penetration and absorption on the hair to produce a color (e.g., a hair manicure), a temporary hair dye in which a colored film containing a colorant (mainly a pigment) is formed on the hair to produce a color (e.g., a hair mascara), and so on. Of these, the temporary hair dye is preferred by consumers as a hair dyeing material that can be freely enjoyed because it has less damage to the hair, good removability by shampooing, and convenience of use. As a conventional temporary hair dye, a temporary hair dye composition containing a film-forming polymer is known in order to fix a colorant as a colored film onto the hair, impart water resistance to this colored film, and prevent color transfer to the skin or clothing.
As the conventional temporary hair dye, one for the purpose of achieving gray hair dyeing is the mainstream, and from the viewpoint of making it easy to apply to areas of concern, such as hairline, ones of a mascara type are known as a product form.
For example, as a temporary hair dye which while containing black titanium oxide with higher safety, makes it possible to achieve hair dyeing with sufficiently small color unevenness and discoloration, is sufficiently low in secondary staining, and is capable of achieving usability and use feel with a good balance, PTL 1 discloses a temporary hair dye containing 35 to 80% by mass of ethanol, black titanium oxide, a (methacryloyloxyethyl carboxybetaine/alkyl methacrylate) copolymer, hydroxypropyl cellulose, and glycerin. It is described that according to the foregoing temporary hair dye, scattering of the liquid can be sufficiently reduced even when using a brush, etc. as an applicator, and thus, it is especially suitable as a temporary hair dye of a mascara type; and also that when applying the hair dye to a target site of the hair, its viscosity is preferably 50 to 30,000 mPa·s.
In addition, as an applicator for the purpose of applying a liquid cosmetic preparation efficiently to the hair or to a narrow range that is an applied area, PTLs 2 and 3 disclose a cosmetic preparation applicator including an application portion provided with an application member formed by an elastic material capable of being impregnated with a liquid cosmetic preparation and a plurality of comb teeth, and having a predetermined shape.

Citation List

Patent Literature

PTL 1: JP 2008-63251 A
PTL 2: JP 2012-232104 A
PTL 3: JP 2018-140018 A

Summary of the Invention

The present invention relates to the following.

[1] A cosmetic for hair including a container having a liquid hair cosmetic preparation accommodated in the inside thereof; and an applicator provided with not only an application body at one end of an application shaft but also a container lid part at the other end of the application shaft, wherein the hair cosmetic preparation has a viscosity at 25°C of 5,000 mPa·s or less; and the application body is provided with an absorptive member having a density of 15 $kg/m^3$ or more and 95 $kg/m^3$ or less, and the absorptive member covers the entirety of the one end of the application shaft.
[2] A cosmetic for hair including a container having a liquid hair cosmetic preparation accommodated in the inside thereof; and an applicator provided with not only an application body at one end of an application shaft but also a container lid part at the other end of the application shaft, wherein the hair cosmetic preparation has a viscosity at 25°C of 5,000 mPa·s or less; and the application body is composed of an absorptive member having a density of 15 $kg/m^3$ or more and 95 $kg/m^3$ or less, and the application body covers the entirety of the one end of the application shaft.

Brief Description of the Drawings

Fig. 1 is an overall view showing a state in which an applicator composing a cosmetic for hair according to an embodiment of the present invention is installed in a container.
Fig. 2 is a longitudinal sectional view showing a state in which the applicator composing the cosmetic for hair according to the embodiment of the present invention is installed in the container.
Fig. 3 is a longitudinal sectional view of the applicator composing a cosmetic for hair according to the embodiment of the present invention.
Fig. 4 is a perspective view of the applicator composing the cosmetic for hair according to the embodiment of the present invention.
Fig. 5 is a longitudinal sectional view of the container composing the cosmetic for hair according to the embodiment of the present invention.
Fig. 6 is a side view of a test applicator 21.
Fig. 7 is a perspective view of the test applicator 21 and a wiping member 81.
Fig. 8 is a side view of a test applicator 22 for comparison as seen from an application body-covered surface of an application shaft.
Fig. 9 is a perspective view of the test applicator 22 for comparison and a wiping member 82.

Detailed Description of the Invention

[Cosmetic for hair]

A cosmetic for hair of the present invention is one including a container having a liquid hair cosmetic preparation accommodated in the inside thereof; and an applicator provided with not only an application body at one end of an application shaft but also a container lid part at the other end of the application shaft, wherein the hair cosmetic preparation has a viscosity at 25°C of 5,000 mPa·s or less; and the application body is provided with an absorptive member having a density of 15 kg/$m^3$ or more and 95 kg/$m^3$ or less, and the absorptive member covers the entirety of the one end of the application shaft. Preferably, the cosmetic for hair of the present invention is one including a container having a liquid hair cosmetic preparation accommodated in the inside thereof; and an applicator provided with not only an application body at one end of an application shaft but also a container lid part at the other end of the application shaft, wherein the hair cosmetic preparation has a viscosity at 25°C of 5,000 mPa·s or less; and the application body is composed of an absorptive member having a density of 15 kg/$m^3$ or more and 95 kg/$m^3$ or less, and the application body covers the entirety of the one end of the application shaft.
In view of the fact that the cosmetic for hair of the present invention has the aforementioned configuration, during applying a liquid hair cosmetic preparation to the hair, it hardly causes liquid dripping or liquid outage and can be evenly applied in a single operation to the hair from the root to the tip. From this fact, the cosmetic for hair of the present invention is suitable as a cosmetic for hair of a mascara type.
Examples of the cosmetic for hair of a mascara type include a hair dye cosmetic article, such as a hair mascara, and an eyelashes makeup cosmetic article, such as an eyelashes mascara. Above all, the cosmetic for hair of the present invention is suitable as a temporary hair dye cosmetic article of a mascara type.
Among temporary hair dyes, there are one for the purpose of achieving gray hair dyeing and one imparting a brilliant color to the hair for the purpose of improving the fashionability. The hair dye for gray hair dyeing is used mainly upon being applied to the hairline. On the other hand, as for the temporary hair dye for the purpose of improving the fashionability, there is also supposed a use mode upon being partially applied onto the hair surface to dye the hair in a mesh state. The temporary hair dye in such a use mode is demanded such that it can be evenly applied in a single operation (one stroke) to the hair from the root to the tip even against long hairs. If the temporary hair dye is reduced in viscosity, though it may be considered that spreading on the hair becomes favorable, it is liable to cause liquid dripping from the applicator or the hair after application, so that there may be generated a problem from the standpoint of handling properties and finishing.
The present invention relates to a cosmetic for hair which is suitable as a cosmetic for hair of a mascara type, especially as a temporary hair dye cosmetic article and which hardly causes liquid dripping or liquid outage during an application operation and can be evenly applied in a single operation to the hair from the root to the tip.
The present inventors have found that a cosmetic for hair in which a liquid hair cosmetic preparation having a viscosity falling within a predetermined range is combined with an applicator satisfying predetermined conditions satisfies the aforementioned requirements.
In accordance with the present invention, it is possible to provide a cosmetic for hair which is suitable as a

cosmetic for hair of a mascara type, especially as a temporary hair dye cosmetic article and hardly causes liquid dripping or liquid outage during an application operation and can be evenly applied in a single operation to the hair from the root to the tip even against long hairs.

The configuration of the cosmetic for hair of the present invention is described by reference to the drawings.

Fig. 1 is an overall view showing a state in which the applicator composing the cosmetic for hair according to an embodiment of the present invention is installed in the container; Fig. 2 is a longitudinal sectional view of the same; Fig. 3 is a longitudinal sectional view of the applicator; Fig. 4 is a perspective view of the same; and Fig. 5 is a longitudinal sectional view of the container.

The cosmetic for hair of the present invention is one that is used by applying a liquid hair cosmetic preparation, such as a temporary hair dye, having been accommodated in the inside of the container, to the hair by using the applicator.

<Container and Applicator>

As shown in Figs. 1 and 2, a cosmetic for hair 100 includes a container 1 having a liquid hair cosmetic preparation L accommodated in the inside thereof and an applicator 2. In an embodiment shown in Figs. 1 and 2, the side of an application body 4 of an application shaft 3 of the applicator 2 is inserted into the container 1 in the cosmetic for hair 100, and the application body 4 is immersed in the liquid hair cosmetic preparation L accommodated in the inside of the container 1.

As shown in Figs. 2 to 4, the applicator 2 is provided with not only the application body 4 at one end of the application shaft 3 but also a container lid part 5 at the other end of the application shaft 3. The application body 4 is a member for bringing the liquid hair cosmetic preparation L in an immersed state into contact with the hair and applying it.

The application shaft 3 may have an approximately pillar or approximately cylindrical shaft part extending toward the direction of the application body 4 from the container lid part 5. Although the cross-sectional shape of the shaft part of the application shaft 3 is not particularly restricted, the shaft part which the application shaft 3 has is preferably in an approximately columnar form from the viewpoint of holding the application body 4 rotatably in the peripheral direction of the application shaft 3, as mentioned later.

Although the material of the application shaft 3 is not particularly limited, a plastic, such as polyethylene and polypropylene, is preferred from the viewpoint of molding processability and light weight.

The application body 4 is one provided with an absorptive member having a density of 15 $kg/m^3$ or more and 95 $kg/m^3$ or less. The application body 4 may be one composed of an absorptive member or may be one in which comb teeth, a brush, or the like is partly provided in addition to the absorptive member. From the viewpoint that the liquid hair cosmetic preparation hardly causes liquid dripping or liquid outage during an application to the hair and is evenly applied in a single operation to the hair from the root to the tip, a coverage ratio of the application body 4 by the aforementioned absorptive member is preferably 50% or more, more preferably 70% or more, still more preferably 90% or more, and yet still more preferably 100% of the outer surface of the application body 4.

From the viewpoint that the liquid hair cosmetic preparation hardly causes liquid dripping or liquid outage during the application to the hair and is evenly applied in a single operation to the hair from the root to the tip, the application body 4 is preferably composed of an absorptive member.

In this specification, the absorptive member refers to a member capable of impregnating a liquid, such as the liquid hair cosmetic preparation L, and sustaining it, and as the form of a representative absorptive member, there is exemplified a sponge.

In the present invention, when the density of the absorptive member with which the application body 4 provided in the applicator 2 is provided falls within the aforementioned range, the liquid hair cosmetic preparation is favorable in sustainability and applicability and can be evenly applied in a single operation to the hair from the root to the tip.

From the viewpoint of sustainability of the liquid hair cosmetic preparation, the density of the absorptive member is 15 $kg/m^3$ or more, preferably 20 $kg/m^3$ or more, more preferably 22 $kg/m^3$ or more, and still more preferably 25 $kg/m^3$ or more. In addition, from the viewpoint that at the time of application operation of a hair cosmetic preparation of a relatively low viscosity, liquid dripping is hardly caused, and the application is evenly achieved, the density of the absorptive member is 95 $kg/m^3$ or less, preferably 93 $kg/m^3$ or less, and more preferably 90 $kg/m^3$ or less. A specific range of the density of the absorptive member is 15 to 95 $kg/m^3$, preferably 20 to 95 $kg/m^3$, more preferably 22 to 93 $kg/m^3$, and still more preferably 25 to 90 $kg/m^3$. With respect to the density as referred to herein, a rectangular parallelepiped is cut out from the absorptive member and measured for its weight W (kg) and lengths of three sides: L (m) in the longitudinal direction, M (m) in the machine direction, and H (m) in the height direction, and the density is calculated according to the following equation.

$$\text{Density } (kg/m^3) = W/(L \times M \times H)$$

In the case of impregnating the absorptive member with a liquid, such as the hair cosmetic preparation, since it occasionally absorbs the liquid hair cosmetic preparation and is swollen, the aforementioned density of the absorptive member is measured using an absorptive member in a dry state. Hereinafter, it should be construed that an outer diameter, an inner diameter, a thickness, and a length of the absorptive member covering the entirety at one end of the application shaft 3 are similarly measured using an absorptive member in a dry state.

Here, the outer diameter of the absorptive member means a length of the longest straight line among straight lines which in a cross section of the absorptive member perpendicular to the length direction of the application shaft (hereinafter also referred to as "application shaft direction"), pass through a center of gravity thereof and have both ends in the outer edge of the cross section, and is expressed with "d1" in Fig. 3. For example, when the shape of the outer periphery in the cross section perpendicular to the application shaft direction of the absorptive member is a perfect circle, the outer diameter means a diameter of the perfect circle.

The inner diameter of the absorptive member means twice the shortest distance from the center of gravity of the absorptive member in the cross section perpendicular to the application shaft direction to the inner surface of the absorptive member, and is expressed with "d2" in Fig. 3. For example, when the shape of the inner periphery in the cross section perpendicular to the application shaft direction is a perfect circle, the inner diameter of the absorptive member means a diameter of the perfect circle.

The thickness of the absorptive member means [{(outer diameter of absorptive member) - (inner diameter of absorptive member)}/2].

The length of the absorptive member means a length of the absorptive member in the application shaft direction and a length of a portion capable of coming into contact with the hair, and is expressed with "h" in Fig. 3.

The outer diameter of the absorptive member can be set to a variety of desired outer diameters to what extent is designed the size of the cosmetic for hair. From the viewpoint that conveyance of the cosmetic for hair is easy, and the hair cosmetic preparation is made easily applicable to the hair, the outer diameter of the absorptive member is preferably 5 mm or more, more preferably 8 mm or more, and still more preferably 10 mm or more, and preferably 35 mm or less, more preferably 25 mm or less, and still more preferably 15 mm or less. A specific range of the outer diameter of the absorptive member is preferably 5 to 35 mm, more preferably 8 to 25 mm, and still more preferably 10 to 15 mm.

From the viewpoint that the liquid sustainability of the liquid hair cosmetic preparation is favorable, and the liquid dripping at the time of use is suppressed, the thickness of the absorptive member is preferably 0.5 mm or more, more preferably 1 mm or more, and still more preferably 1.5 mm or more, and preferably 8 mm or less, more preferably 6 mm or less, and still more preferably 4 mm or less. A specific range of the thickness of the absorptive member is preferably 0.5 to 8 mm, more preferably 1 to 6 mm, and still more preferably 1.5 to 4 mm.

The length of the absorptive member in the application shaft direction can be set to a variety of desired lengths to what extent is designed the width at which the hair cosmetic preparation can be applied in a single operation. From the viewpoint of exhibiting the liquid sustainability of the liquid hair cosmetic preparation and making the applicability of the hair cosmetic preparation favorable, the length of the absorptive member in the application shaft direction is preferably 10 mm or more, more preferably 15 mm or more, and still more preferably 20 mm or more, and preferably 60 mm or less, more preferably 50 mm or less, and still more preferably 40 mm or less. A specific range of the length of the absorptive member in the application shaft direction is preferably 10 to 60 mm, more preferably 15 to 50 mm, and still more preferably 20 to 40 mm.

Although the material of the absorptive member is not particularly restricted so long as the density falls within the aforementioned range, and the liquid hair cosmetic preparation L can be impregnated and sustained, the material of the absorptive member is preferably polyurethane from the viewpoint of exhibiting the liquid sustainability of the liquid hair cosmetic preparation and elasticity suited for application, such as compressibility and restorability. That is, the absorptive member is preferably a sponge made of polyurethane, such as foamed polyurethane.

The application body 4 shown in Figs. 2 to 4 is concerned with an embodiment composed of the aforementioned absorptive member. As shown in Figs. 2 to 4, not only the application body 4 composed of the aforementioned absorptive member is provided at one end of the application shaft 3, but also the absorptive member in the application body 4 covers the entirety of the one end of the application shaft 3. The "entirety of the one end of the application shaft" means the whole of the side face of the application shaft at the one end of the application shaft. That is, at the one end of the application shaft 3, the absorptive member is not concerned with an embodiment of covering only a part of the side face of the application shaft 3 but covers the whole of the side face at the one end of the application shaft 3.

In view of the fact that the absorptive member is composed so as to cover the entirety of the one end of the application shaft 3, a holding amount of the liquid hair cosmetic preparation and an area at which the liquid hair cosmetic preparation is able to come into contact with the hair can be increased. Therefore, it becomes possible to evenly apply the liquid hair cosmetic preparation in a single operation without causing liquid outage on the way of application. In particular, in the case of applying the hair cosmetic preparation to long hairs so as to draw a long and narrow line from the root to the tip of the hair, as compared with the use of a platy application body, the use of the application body of the present embodiment is advantageous from the standpoint that since it is possible to achieve the application to a

narrow region, not only the liquid cosmetic preparation is easily applied to a target site, but also it can be evenly applied in a single operation without causing liquid outage.

For examples, in the case where the application shaft 3 has an approximately columnar shaft part extending toward the direction of the application body 4, the application body 4 can be one provided with an approximately cylindrical absorptive member covering the entirety of the one end of the application shift 3.

It is preferred that the application body 4 is held rotatably in the peripheral direction of the application shaft 3 at the one end of the application shaft 3. For example, in the case where the application shaft 3 has an approximately columnar shaft part, the peripheral direction of the application shaft means its circumferential direction.

When the application body 4 is held rotatably in the peripheral direction of the application shaft 3, on the occasion of applying the hair cosmetic preparation, by pushing the application body 4 against the hair surface and moving the applicator 2 to the direction approximately orthogonal to the shaft direction of the application shaft 3, the hair cosmetic preparation can be applied while rotating the application body 4 to the peripheral direction of the application shaft 3. According to this embodiment, since a contact area between the surface of the application body 4 provided with the absorptive member having the liquid hair cosmetic preparation L impregnated therein and the hair surface is increased, the liquid outage hardly occurs during the application operation, and the effect for enabling it to evenly apply in a single operation is more improved.

Examples of the embodiment of holding the application body 4 rotatably in the peripheral direction of the application shaft 3 include an embodiment in which as shown in Figs. 2 and 3, a rotation body 6 formed of a non-absorptive member, which is held rotatably in the peripheral direction of the application shaft 3, is provided on an outer surface of the application shaft 3, and the application body 4 is provided on an outer surface of the rotation body 6 and at the side of one end of the application shaft 3. Similar to the application shaft 3 and the container lid part 5, the non-absorptive member constituting the rotation body 6 is preferably a plastic, such as polyethylene and polypropylene.

The rotation body 6 shown in Figs. 2 and 3 has an approximately cylindrical shape and is provided so as to cover at least the outer periphery of the one end of the application shaft 3, and it is held in a rotatable state in the peripheral direction of the application shaft 3 at the other end of the application shaft 3, namely the side of the container lid part 5. By allowing the application body 4 provided with an approximately cylindrical absorptive member to engage with the outer surface side of this rotation body 6 from the side of the one end of the application shaft 3, the application body 4 is held rotatably in the peripheral direction of the application shaft 3.

Furthermore, in order to prevent falling of the application body 4 engaged with the rotation body 6 from occurring, it is preferred to hold the application body 4 in the rotation body 6 by using an application body holder 7. The application body holder 7 shown in Figs. 2 to 4 has a protrusion on one face of a disc-like member, and the protrusion is allowed to engage from the side of an end 3a (not illustrated) of one end of the application shaft 3 and held such that the application body 4 does not fall. An outer diameter of the disc-like member can be appropriately selected within a range where not only the application body 4 can be held, but also the application operation of the applicator is not obstructed.

The container lid part 5 is provided at the other end of the application shaft 3 and has both roles as a lid of the container 1 and a site where a user grasps during use of the applicator 2. The container lid part 5 shown in Fig. 5 has an approximately cylindrical shape having an opening at one side, and in the inner circumference in the vicinity of this opening, a female screw 5a capable of performing screw connection with a male screw 1a of the container 1 as shown in Fig. 5 is formed. By fitting the application shaft 3 from the opening of the container lid part 5, the container lid part 5 and the application shaft 3 are engaged with each other in a non-rotatable manner in the peripheral direction.

Although the material of the container lid part 5 is not particularly limited, similar to the application shaft 3, a plastic, such as polyethylene and polypropylene, is preferred from the viewpoint of molding processability and light weight.

On the other hand, though the container 1 is not particularly limited with respect to the shape and the material so long as the liquid hair cosmetic preparation L having a viscosity falling within the aforementioned range can be accommodated in the inside thereof, it preferably has a bottle shape. The material of the container 1 may be the same as or different from that in the application shaft 3 and the container lid part 5, similar to the application shaft 3 and the container lid part 5, a plastic, such as polyethylene and polypropylene, is preferred.

As shown in Figs. 2 and 5, it is preferred that the container 1 not only accommodates the liquid hair cosmetic preparation L but also is provided with a wiping member 8 within a neck part of the container 1. The wiping member 8 is formed of a soft material, such as a rubber, appears an approximately bottomed cylindrical shape, and extends downward from the neck part of the container 1. In the bottom of the wiping member 8, for example, a slit (cut) 8a in a cross shape is formed, and at a position in an upper portion from the bottom and a lower portion from the neck part of the container 1, a disc-like wiping part 8b is provided overhanging inward. In the approximately center of the wiping part 8b, a wiping hole 8c having, for example, a circular shape, through which the application shaft 3 and the application body 4 pass, is formed.

In accordance with the cosmetic for hair 100 having such a configuration, in a state of installing the applicator 2 in the container 1, the application body 4 is dipped in the liquid hair cosmetic preparation L. In addition, in this state, the application shaft 3 passes through the wiping hole 8c and the slit 8a, and the slit 8a is in a state of being rolled downward.

The user holds the container lid part 5, unscrews the female screw 5a of the container lid part 5 and the male screw 1a of the container 1, and then takes out the applicator 2 from the container 1. At this time, the excessive hair cosmetic preparation L attached to the application body 4 of the applicator 2 is wiped off from the internal circumference of the wiping part 8b forming the wiping hole 8c and also wiped off by the slit 8a.

On the occasion of taking out the applicator 2 from the container 1, rolling of the rolled cross-shaped slit 8a returns to the original state, and the slit 8a becomes in an approximately closed state of the bottom. In consequence, when the applicator 2 is taken out from the container 1 and provided for application, even if the container 1 is inadvertently fallen down, the liquid hair cosmetic preparation L is interrupted by the closed slit 8a, whereby flowing out of the liquid hair cosmetic preparation L from the container 1 can be prevented from occurring.

Furthermore, as shown in Fig. 2, it is preferred that the container 1 accommodates stirring balls 9 together with the liquid hair cosmetic preparation L. Even in the case where the blending components, such as a colorant, precipitate in the hair cosmetic preparation, when the user shakes the container 1 by hand, etc., the blending components can be efficiently stirred and uniformly redispersed by the stirring balls 9. The material of the stirring balls 9 is preferably one having a high specific gravity and being hardly worn away, and examples thereof include SUS.

<Hair Cosmetic Preparation>

The hair cosmetic preparation L to be accommodated in the container 1 has a viscosity at 25°C of 5,000 mPa·s or less. By not only allowing the viscosity of the hair cosmetic preparation to be accommodated in the container 1 to fall within the aforementioned range but also using the aforementioned applicator 2, the cosmetic for hair of the present invention becomes a cosmetic for hair in which during the application operation, the hair cosmetic preparation hardly causes liquid dripping or liquid outage and can be evenly applied in a single operation to the hair from the root to the tip.

The viscosity at 25°C of the hair cosmetic preparation may be more than 0 mPa·s. The foregoing viscosity is preferably 1 mPa·s or more, more preferably 5 mPa·s or more, and still more preferably 10 mPa·s or more from the viewpoint of suppressing liquid dripping and splash of liquid during the application operation, and it is preferably 3,000 mPa·s or less, more preferably 2,000 mPa·s or less, still more preferably 1,500 mPa·s or less, yet still more preferably 1,000 mPa·s or less, even yet still more preferably 800 mPa·s or less, even still more preferably 500 mPa·s or less, even still further preferably 300 mPa·s or less, and even yet still more further preferably 150 mPa·s or less from the viewpoint of increasing the holding amount on the application body and evenly applying in a single operation from the root to the tip of the hair without causing liquid outage. A specific range of the viscosity at 25°C of the hair cosmetic preparation is 5,000 mPa·s or less, preferably more than 0 mPa·s and 5,000 mPa·s or less, more preferably 1 to 3,000 mPa·s, still more preferably 1 to 2,000 mPa·s, yet still more preferably 5 to 1,500 mPa·s, even yet still more preferably 5 to 1,000 mPa·s, even still more preferably 5 to 800 mPa·s, even still further preferably 5 to 500 mPa·s, even still further preferably 5 to 300 mPa·s, even yet still more further preferably 10 to 300 mPa·s, and even yet still more further preferably 10 to 150 mPa·s.

The aforementioned viscosity is a value when measured at a temperature of 25±1°C for 1 minute at a predetermined rotation rate with a B-type viscometer using a rotor according to the measuring adaptive range. Specifically, 70 to 80 g of the hair cosmetic preparation is charged in a screw tube having a capacity of 100 to 200 mL and shaken by hand 100 times in a width of about 30 cm for 30 seconds such that it becomes uniform; further, immediately after standing up 30 times at a speed of one time per second, using a B-type viscosity (TV-10M, available from Toki Sangyo Co., Ltd.), the viscosity is measured under conditions of using a rotor No. M1 for up to viscosities of 100 mPa·s at a rotation speed of 100 rpm, using a rotor No. M2 in a viscosity range of 100 to 500 mPa·s at a rotation speed of 60 rpm, using a rotor No. M3 in a viscosity range of 500 to 2,000 mPa·s at a rotation speed of 60 rpm, and using a rotor No. M4 in a viscosity range of 2,000 to 10,000 mPa·s at a rotation speed of 60 rpm. In more detail, the viscosity can be measured by the method described in the section of Examples.

The hair cosmetic preparation constituting the cosmetic for hair of the present invention is preferably a hair dye, and more preferably a temporary hair dye. In the case where the hair cosmetic preparation is a hair dye, particularly a temporary hair dye, the hair cosmetic preparation preferably contains the following components (A) to (C):

(A) Colorant
(B) Film-forming polymer having a silicone structure
(C) Nonaqueous solvent

(Component (A): Colorant)

In the case where the hair cosmetic preparation is a hair dye, the colorant that is the component (A) is used for the purpose of dyeing the hair in a desired color shade.

Examples of the colorant include a pigment and a dye, and these may be used in combination. In particular,

from the viewpoint of forming a temporary hair dye for imparting a brilliant color to the hair, the component (A) is preferably a pigment. As the pigment, all of an inorganic pigment and an organic pigment can be used, and these can also be used in combination.

The component (A) which is used in the present invention preferably contains an interference pearl pigment (A1) and a colorant (A2) other than the interference pearl pigment. When the component (A) is a constitution containing the component (A1) and the component (A2), in the case where the hair cosmetic preparation is a temporary hair dye, particularly a temporary hair dye imparting a brilliant color to the hair for the purpose of improving the fashionability, a color-developing behavior is hardly influenced by a base hair color, so that a fixed hair dyeing effect can be imparted. With respect to the wording "color-developing behavior is hardly influenced by a base hair color, so that a fixed hair dyeing effect is imparted" as referred to herein, in detail, it is meant that regardless of whether the base hair color is a bright color or a dark color, a color change is produced according to the applied color without significantly changing the brightness (lightness) of the hair before and after hair dyeing.

In hair dyes, such as a temporary hair dye, a pigment, etc. is generally used as a colorant. However, for example, in a temporary hair dye using only an organic pigment as the colorant, when dyeing the hair with a dark color, such as black hair, since a color change is hardly seen, in order to provide sufficient color development, it was needed to blend an organic pigment in a considerable amount. On the other hand, in a temporary hair dye having a considerable amount of an organic pigment blended therein, when applied on the hair with a bright color, such as bleached hair, the color development was too gaudy, so that such was not practically useful.

As a result of extensive and intensive investigations made by the present inventors, it has been found that by using the interference pearl pigment that is the component (A1) and the colorant other than the component (A1) (component A2)), the color-developing behavior is hardly influenced by a base hair color, so that a fixed hair dyeing effect can be imparted.

The interference pearl pigment that is the component (A1) is a pigment exhibiting a pearly luster owing to a reflection and interference action of light and refers to one having a coating layer on a surface of a core particle, the coating layer including a reflection and interference layer and having translucency. The core particle is typically a semi-transparent tabular particle, and the reflection and interference layer is constituted of a colorless metal oxide, such as titanium dioxide, or a colorless metal. In addition, though the coating layer occasionally exhibits a wavelength color originated from its reflected light depending upon the thickness of the reflection and interference layer included therein, the coating layer per se has translucency.

In consequence, even when the component (A1) is singly applied on the hair with a bright color, the color change is hardly seen. However, when it is applied on the hair with a dark color, not only a glittering luster is provided owing to a reflection and interference action of light, but also a color change is readily seen. Accordingly, when using the component (A1), even if a large amount of a colorant that is the component (A2), such as an organic pigment, is not blended, it may be considered that when applying on the hair with a dark color, a temporary hair dye exhibiting sufficient color development is obtained. In addition, it may be considered that when applying the foregoing temporary hair dye on the hair with a bright color, desired color development can be imparted without significantly changing the brightness of hair. It may be considered that according to the foregoing mechanism of action, the color-developing behavior is hardly influenced by a base hair color, so that a fixed hair dyeing effect can be imparted.

[Interference Pearl Pigment (A1)]

As mentioned above, the interference pearl pigment is a pigment exhibiting a pearly luster owing to a reflection and interference action of light and having a coating layer on a surface of a core particle, the coating layer including a reflection and interference layer and having translucency. In the present invention, the coating layer in the interference pearl pigment does not substantially contain a colored metal oxide and a colored metal. It is meant by the wording "does not substantially contain" that the coating layer does not contain any of a colored metal oxide and a colored metal within a range where the translucency of the coating layer per se does not appear. The content of the colored metal oxide and the colored metal in the coating layer is preferably 3% by mass or less, more preferably 1% by mass or less, and still more preferably 0.1% by mass or less. Examples of the colored metal oxide include iron oxide, copper oxide, cobalt oxide, chromium oxide, and nickel oxide, and examples of the colored metal include gold and copper.

In this specification, the interference pearl pigment is distinguished from a colored pearl pigment. The colored pearl pigment is a pigment exhibiting a pearly luster owing to a reflection and interference action of light and in general, has a coating layer on a surface of a core particle, and the foregoing coating layer is colored with the aforementioned colored metal oxide or colored metal and does not have translucency. In consequence, the colored pearl pigment is different from the interference pearl pigment and has concealability.

A pigment having a coating layer on a surface of a core particle, the coating layer including a reflection and interference layer constituted of a colorless metal oxide or a colorless metal and a colored layer formed on a surface of the reflection and interference layer and constituted of an organic pigment is, in general, occasionally named as a

composite pearl pigment. However, in this specification, such a pigment is included in the interference pearl pigment so long as the coating layer has translucency as a whole.

Examples of a material of the core particle constituting the component (A1) include mica, sericite, talc, kaolin, a smectite group clay mineral, bismuth oxychloride, synthetic mica, synthetic sericite, tabular silica, and tabular alumina. Of these, mica is preferred.

The reflection and interference layer included in the coating layer of the component (A1) is preferably a layer formed of a thin film composed mainly of a colorless metal oxide, such as titanium dioxide, zinc oxide, and aluminum oxide, or a colorless metal, such as titanium, zirconium, zinc, tin, silicon, and aluminum, and it may be of a single-layered structure or a multi-layered structure. The colorless metal oxide or colorless metal constituting the reflection and interference layer is more preferably at least one selected from the group consisting of titanium and titanium dioxide, and still more preferably titanium dioxide.

The interference pearl pigment that is the component (A1) is more preferably titanium dioxide-coated mica (micaceous titanium) from the viewpoint of imparting a fixed hair dyeing effect regardless of the influence by a base hair color when used as a temporary hair dye.

Although a ratio between the core particle and the reflection and interference layer constituting the component (A1) is not particularly restricted, a mass ratio of the core particle to the colorless metal oxide and the colorless metal constituting the reflection and interference layer [(core particle)/(colorless metal oxide and colorless metal constituting reflection and interference layer)] is preferably 25/75 to 80/20, more preferably 30/70 to 70/30, and still more preferably 50/50 to 65/35.

An average particle diameter of the component (A1) is preferably 2 pm or more, more preferably 5 pm or more, and still more preferably 10 pm or more, and preferably 40 pm or less, more preferably 35 pm or less, and still more preferably 30 pm or less in terms of a volume median particle diameter (D50). A specific range of the volume median particle diameter (D50) of the component (A1) is preferably 2 to 40 pm, more preferably 5 to 35 pm, and still more preferably 10 to 30 μm. When the D50 of the component (A1) falls within the aforementioned range, during using the hair cosmetic preparation as a temporary hair dye, a fixed hair dyeing effect can be imparted regardless of the influence by a base hair color.

The D50 of the component (A1) is a value measured with a laser diffraction particle size analyzer.

Examples of a commercially available interference pearl pigment which can be used as the component (A1) include "Timiron Super Blue" (titanium mica, mica: 51% by mass, titanium dioxide: 49% by mass, D50: 18 to 25 pm), "Timiron Super Gold" (titanium mica, mica: 60% by mass, titanium dioxide: 40% by mass, D50: 18 to 25 pm), "Timiron Super Green" (titanium mica, mica: 55% by mass, titanium dioxide: 45% by mass, D50: 18 to 25 pm), and "Timiron Super Red" (titanium mica, mica: 52% by mass, titanium dioxide: 48% by mass, D50: 18 to 25 pm), all being available from Merck.

The content of the component (A1) in the hair cosmetic preparation is preferably 1.5% by mass or more, more preferably 2.5% by mass or more, still more preferably 3.0% by mass or more, yet still more preferably 3.5% by mass or more, and even yet still more preferably 4.0% by mass or more, and preferably 10% by mass or less, more preferably 9.5% by mass or less, still more preferably 9.0% by mass or less, and yet still more preferably 8.5% by mass or less from the viewpoint of imparting a fixed hair dyeing effect regardless of the influence by a base hair color in the case where the hair cosmetic preparation is a temporary hair dye. A specific range of the content of the component (A1) in the hair cosmetic preparation is preferably 1.5 to 10% by mass, more preferably 2.5 to 10% by mass, still more preferably 3.0 to 9.5% by mass, yet still more preferably 3.5 to 9.0% by mass, and even yet still more preferably 4.0 to 8.5% by mass.

[Colorant (A2) Other Than Interference Pearl Pigment]

The component (A2) is a colorant other than the component (A1). In view of the fact that the hair cosmetic preparation contains the component (A2), in the case where the hair cosmetic preparation is a temporary hair dye, a desired color shade can be imparted, and by further jointly using the component (A1), the color-developing behavior is hardly influenced by a base hair color, so that a fixed hair dyeing effect can be imparted.

As for the component (A2), any pigment can be used irrespective of the shape (e.g., a spherical shape, an acicular shape, and a tabular shape), the particle diameter, and the particle structure (e.g., a porous structure and a nonporous structure), and examples thereof include an inorganic pigment and an organic pigment other than the component (A1).

Examples of the inorganic pigment other than the component (A1) include inorganic black pigments, such as carbon black, black iron oxide, and black titanium oxide; inorganic red pigments, such as iron oxide (red iron oxide), iron hydroxide, and iron titanate; inorganic brown pigments, such as γ-iron oxide; inorganic yellow pigments, such as yellow iron oxide and ocher; inorganic blue pigments, such as ultramarine blue and Prussian blue; inorganic violet pigments, such as manganese violet and cobalt violet; inorganic green pigments, such as chromium oxide, chromium hydroxide, and cobalt titanate; inorganic white pigments, such as titanium oxide, zinc oxide, cerium oxide, and barium sulfate; metal

powder pigments, such as gold powder, silver powder, copper powder, aluminum powder, and brass powder; and colored pearl pigments, such as iron oxide-coated mica, iron oxide-coated micaceous titanium, black iron oxide-coated mica, black iron oxide-coated micaceous titanium, yellow iron oxide-coated mica, iron oxide/black iron oxide-coated micaceous titanium, iron oxide/Prussian blue-coated micaceous titanium, iron oxide-coated bismuth oxychloride, and iron oxide-coated bismuth oxychloride mica.

Examples of the organic pigment include Red No. 201, Red No. 202, Red No. 203, Red No. 204, Red No. 205, Red No. 206, Red No. 207, Red No. 208, Red No. 219, Red No. 220, Red No. 221, Red No. 226, Red No. 228, Red No. 404, Red No. 405, Orange No. 203, Orange No. 204, Orange No. 401, Yellow No. 205, Yellow No. 401, and Blue No. 404.

As the component (A2) other than those mentioned above, glittering powders, such as a polyethylene terephthalate/aluminum/epoxy laminate and a polyethylene terephthalate/polyolefin laminated film, and various dyes, such as a direct dye, an acid dye, and an edible dye, can also be used.

The aforementioned colorants can be used alone or in appropriate combination of two or more thereof according to the desired color shade.

The content of the component (A2) in the hair cosmetic preparation is preferably 0.5% by mass or more, more preferably 0.8% by mass or more, and still more preferably 1.0% by mass or more from the viewpoint of imparting a desired color shade in the case where the hair cosmetic preparation is a temporary hair dye. In addition, it is preferably 8.0% by mass or less, more preferably 7.0% by mass or less, still more preferably 6.0% by mass or less, yet still more preferably 5.0% by mass or less, and even yet still more preferably 4.0% by mass or less from the viewpoint of imparting a fixed hair dyeing effect regardless of the influence by a base hair color during using as a temporary hair dye. A specific range of the content of the component (A2) in the hair cosmetic preparation of the present invention is preferably 0.5 to 8.0% by mass, more preferably 0.5 to 7.0% by mass, still more preferably 0.8 to 6.0% by mass, yet still more preferably 0.8 to 5.0% by mass, and even yet still more preferably 1.0 to 4.0% by mass.

From the viewpoint of allowing the hair cosmetic preparation to fall within the aforementioned predetermined viscosity range and the viewpoint of improving the hair dyeing effect on the hair with a dark color, such as black hair, in the case where the hair cosmetic preparation is a temporary hair dye, the total content of the component (A1) and the component (A2) in the hair cosmetic preparation is preferably 2.0% by mass or more, more preferably 3.0% by mass or more, still more preferably 3.5% by mass or more, yet still more preferably 4.0% by mass or more, even yet still more preferably 5.0% by mass or more, and even still more preferably 6.0% by mass or more. In addition, the foregoing total content is preferably 12.0% by mass or less, more preferably 11.0% by mass or less, still more preferably 10.0% by mass or less, and yet still more preferably 9.5% by mass or less. A specific range of the total content of the component (A1) and the component (A2) in the hair cosmetic preparation is preferably 2.0 to 12.0% by mass, more preferably 3.0 to 11.0% by mass, still more preferably 3.5 to 11.0% by mass, yet still more preferably 4.0 to 10.0% by mass or more, even yet still more preferably 5.0 to 10.0% by mass, and even still more preferably 6.0 to 9.5% by mass.

In the case where the hair cosmetic preparation is a temporary hair dye, from the viewpoint that a desired color shade can be imparted and that a lightness change on the hair with a bright color, such as bleached hair, is made small, a mass ratio [(A1)/{(A1) + (A2)}] of the component (A1) to the total content of the component (A1) and the compound (A2) is preferably 0.30 or more, more preferably 0.40 or more, still more preferably 0.50 or more, and yet still more preferably 0.60 or more, and preferably 0.96 or less, more preferably 0.94 or less, still more preferably 0.92 or less, and yet still more preferably 0.90 or less. A specific range of the foregoing mass ratio [(A1)/{(A1) + (A2)}] is preferably 0.30 to 0.96, more preferably 0.40 to 0.94, still more preferably 0.50 to 0.92, and yet still more preferably 0.60 to 0.90.

The content of the component (A) in the hair cosmetic preparation may be the same as the aforementioned total content of the component (A1) and the component (A2), and it is preferably 2.0% by mass or more, more preferably 3.0% by mass or more, still more preferably 3.5% by mass or more, yet still more preferably 4.0% by mass or more, even yet still more preferably 5.0% by mass or more, and even still more preferably 6.0% by mass or more, and preferably 12.0% by mass or less, more preferably 11.0% by mass or less, still more preferably 10.0% by mass or less, and yet still more preferably 9.5% by mass or less. A specific range of the content of the component (A) in the hair cosmetic preparation is preferably 2.0 to 12.0% by mass, more preferably 3.0 to 11.0% by mass, still more preferably 3.5 to 11.0% by mass, yet still more preferably 4.0 to 10.0% by mass, even yet still more preferably 5.0 to 10.0% by mass, and even still more preferably 6.0 to 9.5% by mass.

(Component (B): Film-Forming Polymer Having Silicone Structure)

The film-forming polymer having a silicone structure that is the component (B) is able to impart abrasion resistance, water resistance, removability by shampooing, softness, high color develop ability, and so on to the hair cosmetic preparation. In addition, in the case where the hair cosmetic preparation is a temporary hair dye, the temporary hair dye can be made such that color transfer to the skin or clothing after hair dyeing is less, and a feeling on the hair is favorable.

The component (B) is a polymer not only having a silicone structure in at least a part thereof but also having

a film-forming ability. The "silicone structure" refers to a structure represented by the following general formula (I).

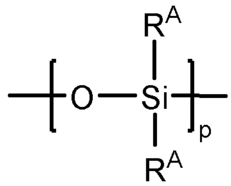

(I)

In the general formula (I), $R^A$'s are each independently a hydrocarbon group having 1 or more and 12 or less carbon atoms; and p is an integer of 1 or more. $R^A$ is preferably an alkyl group having 1 or more and 12 or less carbon atoms or an aryl group having 6 or more and 12 or less carbon atoms, more preferably an alkyl group having 1 or more 12 or less carbon atoms or a phenyl group, still more preferably an alkyl group having 1 or more and 3 or less carbon atoms, and yet still more preferably a methyl group.

Although the silicone structure may be present in any of a main chain and a side chain in the polymer, it is preferably present in a main chain. In the case where the silicone structure is present in a polymer main chain, a binding mode thereof is not particularly restricted, and for example, the silicone structure may be present in an end of the polymer main chain, or may be a copolymerization polymer in which the silicone structure is bound in a block state or a random state in the polymer main chain. In addition, a polymer which has been graft modified with a compound having a silicone structure can also be used.

The component (B) is preferably a poly(N-acylalkyleneimine)-modified organopolysiloxane from the viewpoint that it imparts abrasion resistance, water resistance, removability by shampooing, softness, high color developability, and so on and that in the case where the hair cosmetic preparation is a temporary hair dye, color transfer after hair dyeing is less, and a feeling on the hair is made favorable. Specifically, the poly(N-acylalkyleneimine)-modified organopolysiloxane is an organopolysiloxane in which a poly(N-acylalkyleneimine) segment composed of a repeating unit represented by the following general formula (1) is bound to at least two silicon atoms of an organopolysiloxane segment constituting the main chain via a hetero atom-containing alkylene group.

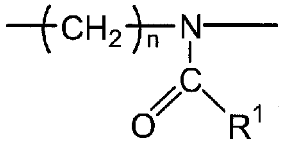

(1)

In the formula, $R^1$ represents a hydrogen atom, an alkyl group having 1 or more and 22 or less carbon atoms, an aralkyl group having 7 or more and 22 or less carbon atoms, or an aryl group having 6 or more and 22 or less carbon atoms; and n represents 2 or 3.

Although the poly(N-acylalkyleneimine) segment can be bound to at least two arbitrary silicon atoms constituting the organopolysiloxane segment via a hetero atom-containing alkylene group, it is preferably bound to at least one silicon atom excluding the both ends via the aforementioned alkylene group, and more preferably bound to at least two silicon atoms excluding the both ends via the aforementioned alkylene group.

Examples of the hetero atom-containing alkylene group mediating in the binding between the organopolysiloxane segment and the poly(N-acylalkyleneimine) include an alkylene group containing 1 to 3 of a nitrogen atom, an oxygen atom, and/or a sulfur atom and having 2 or more and 20 or less carbon atoms. Specific examples thereof include the following groups.

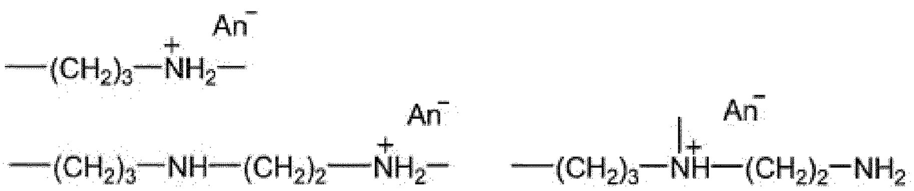

$$-(CH_2)_3-S-$$

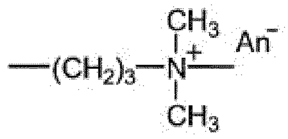

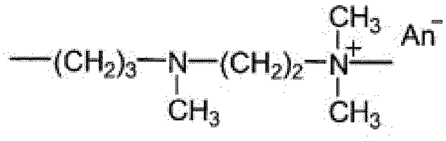

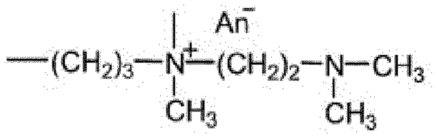

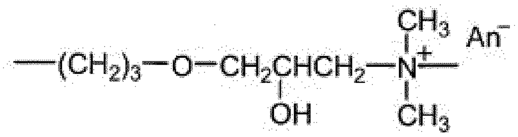

In the formulae, $An^-$ represents an anion.

The N-acylalkyleneimine unit constituting the poly(N-acylalkyleneimine) segment is one represented by the general formula (1).

Examples of the alkyl group having 1 or more and 22 or less carbon atom in $R^1$ of the general formula (1) include linear, branched, or cyclic alkyl groups having 1 or more and 22 or less carbon atoms, and specifically, examples thereof include a methyl group, an ethyl group, a n-propyl group, an isopropyl group, a n-butyl group, a tert-butyl group, a pentyl group, a hexyl group, a cyclohexyl group, a heptyl group, an octyl group, a nonyl group, a decyl group, an undecyl group, a dodecyl group, an octadecyl group, a nonadecyl group, an eicosyl group, and a docosyl group. Of these, an alkyl group having 1 or more and 10 or less carbon atoms is preferred, an alkyl group having 1 or more and 6 or less carbon atoms is more preferred, and an alkyl group having 1 or more and 4 or less carbon atoms is still more preferred.

Examples of the aralkyl group having 7 or more and 22 or less carbon atoms in $R^1$ of the general formula (1) include a benzyl group, a phenethyl group, a trityl group, a naphthylmethyl group, and an anthracenylmethyl group. Of these, an aralkyl group having 7 or more and 14 or less carbon atoms is preferred, and an aralkyl group having 7 or more and 10 or less carbon atoms is more preferred.

Examples of the aryl group having 6 or more and 22 or less carbon atoms in $R^1$ of the general formula (1) include a phenyl group, a tolyl group, a xylyl group, a naphthyl group, a biphenyl group, an anthryl group, and a phenanthryl group. Of these, an aryl group having 6 or more and 12 or less carbon atoms is preferred, and an aryl group having 6 or more and 9 or less carbon atoms is more preferred.

$R^1$ is preferably an alkyl group having 1 or more and 22 or less carbon atoms, more preferably an alkyl group having 1 or more and 10 or less carbon atoms, still more preferably an alkyl group having 1 or more and 6 or less carbon atoms, and still more preferably an alkyl group having 1 or more and 4 or less carbon atoms.

Examples of the preferred poly(N-acylalkyleneimine)-modified organosiloxane include a poly(N-formylethyleneimine)organosiloxane, a poly(N-acetylethyleneimine)organosiloxane, and a poly(N-propionylethyleneimine)organosiloxane, and these can be used alone or in combination of two or more thereof.

Among those mentioned above, a poly(N-propionylethyleneimine)organosiloxane is preferred, and poly(N-propionylethyleneimine)methylsiloxane (N-propionylpolyethyleneimine/methylpolysiloxane copolymer; polysilicone-9) is more preferred from the viewpoint that it imparts abrasion resistance, water resistance, removability by shampooing, softness, high color developability, and so on and that in the case where the hair cosmetic preparation is a temporary hair dye, color transfer after hair dyeing is less, and a feeling on the hair is made favorable.

The content of the component (B) in the hair cosmetic preparation is preferably 1% by mass or more, more preferably 2% by mass or more, still more preferably 3% by mass or more, and yet still more preferably 4% by mass or more from the viewpoint that it imparts abrasion resistance, water resistance, removability by shampooing, softness, high color developability, and so on and that in the case where the hair cosmetic preparation is a temporary hair dye, color transfer after hair dyeing is less. In addition, the content of the component (B) in the hair cosmetic preparation is preferably 30% by mass or less, more preferably 20% by mass or less, still more preferably 15% by mass or less, and yet still more preferably 12% by mass or less from the viewpoint of allowing the hair cosmetic preparation to fall within the aforementioned predetermined viscosity range and the viewpoint of improving a feeling on the hair after hair dyeing in the case where the hair cosmetic preparation is a temporary hair dye. A specific range of the content of the component (B) in the hair cosmetic preparation is preferably 1 to 30% by mass, more preferably 2 to 20% by mass, still more

preferably 3 to 15% by mass, and yet still more preferably 4 to 12% by mass.

In the hair cosmetic preparation, a mass ratio [(B)/(A)] of the component (B) to the component (A) is preferably 0.2 or more, more preferably 0.5 or more, and still more preferably 0.8 or more from the viewpoint that in the case where the hair cosmetic preparation is a temporary hair dye, color transfer after hair dyeing is less, and it is preferably 3 or less, more preferably 1.4 or less, and still more preferably 1.2 or less from the viewpoint of improving a feeling on the hair after hair dyeing in the case where the hair cosmetic preparation is a temporary hair dye. A specific range of the mass ratio [(B)/(A}] is preferably 0.2 to 3, more preferably 0.5 to 1.4, and still more preferably 0.8 to 1.2.

In the case of using the component (A1), a mass ratio [(B)/(A1)] of the component (B) to the component (A1) in the hair cosmetic preparation is preferably 0.1 or more, more preferably 0.2 or more, still more preferably 0.5 or more, and yet still more preferably 1 or more from the viewpoint that in the case where the hair cosmetic preparation is a temporary hair dye, color transfer after hair dyeing is less, and it is preferably 10 or less, more preferably 5 or less, still more preferably 3 or less, and yet still more preferably 2 or less from the viewpoint of improving a feeling on the hair after hair dyeing in the case where the hair cosmetic preparation is a temporary hair dye. A specific range of the mass ratio [(B)/(A1)] is preferably 0.1 to 10, more preferably 0.2 to 5, still more preferably 0.5 to 3, and yet still more preferably 1 to 2.

(Component (B)': Polymer Other Than Component (B))

The hair cosmetic preparation in the present invention may further contain a polymer other than the aforementioned component (B) as a component (B)'. By appropriately containing the component (B)', it becomes easy to control the hair cosmetic preparation to the aforementioned predetermined viscosity range.

The component (B)' is not particularly restricted so long as it is a polymer other than the component (B) and is one which is typically used for the hair cosmetic preparation, and examples of its classification include a cationic polymer, an anionic polymer, a nonionic polymer, and an amphoteric polymer other than the component (B).

In the case where the hair cosmetic preparation contains the component (A1), from the viewpoint of suppressing caking of the component (A1) and making redispersibility favorable, it is preferred to use a cationic polymer as the component (B)'.

Since the interference pearl pigment that is the component (A1) is a tabular inorganic powder having a high specific gravity, there is a tendency that it precipitates in the low-viscosity hair cosmetic preparation to accumulate in the bottom of the container and is solidified hard (readily causes hard caking). However, when the hair cosmetic preparation contains a cationic polymer, it forms a bulky soft aggregate due to an electrostatic interaction between the surface of the negatively charged component (A1) and the cationic polymer, and therefore, it may be considered that the redispersion can also be easily achieved through shaking of the container without causing hard caking after sedimentation.

In this specification, the cationic polymer refers to a water-soluble polymer having a cation group or a group capable of being ionized to become a cation group (these groups will be hereinafter also referred to collectively as "cationic group"). The cationic polymer which is used in the present invention is preferably one having favorable dispersibility in a nonaqueous solvent that is the component (C) as mentioned later.

From the viewpoint of suppressing caking and improving redispersibility of the component (A1), a cation charge density of the cationic polymer is preferably 0.05 meq/g or more, more preferably 0.1 meq/g or more, still more preferably 0.2 meq/g or more, yet still more preferably 0.4 meq/g or more, and even yet still more preferably 0.5 meq/g or more, and preferably 6.5 meq/g or less, more preferably 3.8 meq/g or less, still more preferably 2.5 meq/g or less, yet still more preferably 2.0 meq/g or less, and even yet still more preferably 1.5 meq/g or less. A specific range of the cation charge density of the cationic polymer is preferably 0.05 to 6.5 meq/g, more preferably 0.1 to 3.8 meq/g, still more preferably 0.2 to 2.5 meq/g, yet still more preferably 0.4 to 2.0 meq/g, and even yet still more preferably 0.5 to 1.5 meq/g.

The cation charge density of the cationic polymer refers to [(molar number of the cationic group contained per 1 gram of the cationic polymer) $\times$ 1,000 (meq/g)].

The cationic polymer may be used in combination of two or more thereof, and it this case, the cation charge density of the cationic polymer is determined through calculation of a weighted average from the cation charge densities and the blending amounts of the respective cationic polymers.

Specific examples of the cationic polymer include a cationized guar gum (e.g., JAGUAR EXCEL (available from Solvay (Novecare)), a cationized tara gum (e.g., CATINAL CTR-100 (available from Toho Chemical Industry Co., Ltd.)), a cationized locust bean gum (e.g., CATINAL CLB-100 (available from Toho Chemical Industry Co., Ltd.)), a cationized polyvinyl alcohol (e.g., GOHSENX K-434 (available from The Nippon Synthetic Chemical Industry Co., Ltd.)) and CM318 (available from Kuraray Co., Ltd.)), a vinylpyrrolidone/N,N-dimethylaminoethyl methacrylate copolymer diethyl sulfate (e.g., H.C POLYMER IS(M), and H.C POLYMER 2 (both being available from Osaka Organic Chemical Industry Ltd.)), a vinylpyrrolidone/dimethylaminopropyl methacrylamide/lauryl dimethylaminopropyl methacrylamide copolymer (e.g., Styleze W-20 (available from Ashland)), polydimethylmethylenepiperidinium chloride (e.g., Marquat 100 (available from Lubrizol)), a dimethyldiallylammonium chloride/acrylamide copolymer (e.g., Marquat 550 (available from

Lubrizol)), a vinylpyrrolidone/N,N-dimethylaminoethyl methacrylate copolymer diethyl sulfate (e.g., Gafquat 734 (available from Ashland)), a vinylpyrrolidone/N,N-dimethylaminoethyl methacrylate copolymer (e.g., Gafquat 440 (available from Ashland)), an N,N-dimethylaminoethyl methacrylate diethyl sulfate/N,N-dimethyl acrylamide/polyethylene glycol dimethacrylate copolymer (Polyquaternium-52) (e.g., Sofcare KG-101-E, Sofcare KG-101W-E, and Sofcare KG-301P (all being available from Kao Corporation)), ammonium-modified hydroxyethyl cellulose (e.g., a SoftCAT SL-30 polymer (available from The Dow Chemical Company)), and an acrylamide/DMAPA acrylate/methoxy methacrylate PEG copolymer. These can be used alone or in combination of two or more thereof.

Of these, from the viewpoint of suppressing caking and improving redispersibility of the component (A1) and the viewpoint that in the case where the hair cosmetic preparation is a temporary hair dye, color transfer after hair dyeing is less, and a feeling on the hair is improved, the cationic polymer is preferably at least one selected from the group consisting of a dimethyldiallylammonium chloride/acrylamide copolymer, a vinylpyrrolidone/N,N-dimethylaminoethyl methacrylate copolymer diethyl sulfate, an N,N-dimethylaminoethyl methacrylate diethyl sulfate/N,N-dimethyl acrylamide/polyethylene glycol dimethacrylate copolymer (Polyquaternium-52), ammonium-modified hydroxyethyl cellulose, and an acrylamide/DMAPA acrylate/methoxy methacrylate PEG copolymer; more preferably at least one selected from the group consisting of a dimethyldiallylammonium chloride/acrylamide copolymer, a vinylpyrrolidone/N,N-dimethylaminoethyl methacrylate copolymer diethyl sulfate, and an N,N-dimethylaminoethyl methacrylate diethyl sulfate/N,N-dimethyl acrylamide/polyethylene glycol dimethacrylate copolymer (Polyquaternium-52); and still more preferably an N,N-dimethylaminoethyl methacrylate diethyl sulfate/N,N-dimethyl acrylamide/polyethylene glycol dimethacrylate copolymer (Polyquaternium-52).

In the case of using the component (B)' in the hair cosmetic preparation, from the viewpoint of allowing the hair cosmetic preparation to fall within the aforementioned predetermined viscosity range and the viewpoint of suppressing caking and improving redispersibility of the component (A1), the content of the component (B)' in the hair cosmetic preparation is preferably 0.05% by mass or more, more preferably 0.1% by mass or more, and still more preferably 0.2% by mass or more, and preferably 5% by mass or less, more preferably 3% by mass or less, still more preferably 2.5% by mass or less, and yet still more preferably 1% by mass or less. A specific range of the content of the component (B)' in the hair cosmetic preparation is preferably 0.05 to 5% by mass, more preferably 0.1 to 3% by mass, still more preferably 0.1 to 2.5% by mass, and yet still more preferably 0.2 to 1% by mass.

(Component (C): Nonaqueous Solvent)

The nonaqueous solvent that is the component (C) is used for the purpose of dispersing or dissolving the component (A) and the component (B) and other component in the hair cosmetic preparation. The nonaqueous solvent is a solvent other than water, and from the viewpoint of dispersing or dissolving the respective components in the hair cosmetic preparation and the viewpoint of improving applicability and a drying rate after application, a volatile polar solvent is preferably contained.

Examples of the volatile polar solvent include at least one selected from the group consisting of an alcohol, an ester, and a ketone, each having preferably 6 or less carbon atoms, and more preferably 4 or less carbon atoms, and it is preferred to contain an alcohol having 4 or less carbon atoms. Examples of the alcohol having 4 or less carbon atoms include ethanol, 1-propanol, 2-propanol, 1-butanol, and 2-butanol. Among the foregoing alcohols, at least one selected from the group consisting of ethanol and 2-propanol is more preferred, and from the viewpoint of improving a drying rate after application, ethanol is still more preferred.

The content of the at least one selected from the group consisting of ethanol and 2-propanol in the component (C) is preferably 30% by mass or more, more preferably 50% by mass or more, still more preferably 80% by mass or more, and yet still more preferably 85% by mass or more, and an upper limit thereof is 100% by mass from the viewpoint that applicability and a drying rate after application are improved and the viewpoint that in the case where the hair cosmetic preparation is a temporary hair dye, a lightness change on the hair with a dark color, such as black hair, and on the hair with a bright color, such as bleached hair, is made small. In addition, the content of ethanol in the component (C) is preferably 30% by mass or more, more preferably 50% by mass or more, and still more preferably 80% by mass or more, and an upper limit thereof is 100% by mass from the viewpoint that applicability and a drying rate after application are improved and the viewpoint that in the case where the hair cosmetic preparation is a temporary hair dye, a lightness change on the hair with a dark color, such as black hair, and on the hair with a bright color, such as bleached hair, is made small.

The content of the component (C) in the hair cosmetic preparation is preferably 30% by mass or more, more preferably 50% by mass or more, and still more preferably 70% by mass or more, and preferably 97% by mass or less from the viewpoint of controlling the hair cosmetic preparation to the aforementioned predetermined viscosity range, the viewpoint of dispersing or dissolving the respective components in the hair cosmetic preparation, and the viewpoint of improving applicability and a drying rate after application.

From the viewpoint that drying after application is fast, handling properties during use are favorable, and in the

case where the hair cosmetic preparation is a temporary hair dye, a lightness change on the hair with a bright color, such as bleached hair, is made small, the content of water in the hair cosmetic preparation is preferably 40% by mass or less, more preferably 30% by mass or less, still more preferably 20% by mass or less, yet still more preferably 15% by mass or less, yet still more preferably 10% by mass or less, even yet still more preferably 5% by mass or less, even still more further preferably 2% by mass or less, and even yet still more further preferably 1% by mass or less, and a lower limit thereof is 0% by mass.

(Silicone Oil)

From the viewpoint of improving dispersibility of the component (B) and the component (B)' and the viewpoint that in the case where the hair cosmetic preparation is a temporary hair dye, a feeling on the hair after hair dyeing is improved, the hair cosmetic preparation can further contain a silicone oil.

As for the silicone oil, there are preferably exemplified a phenyl-modified silicone and a dimethyl silicone, each of which has a dynamic viscosity at 25°C of 200 $mm^2/g$ or less.

Examples of the phenyl-modified silicone include methylphenyl polysiloxane (diphenyl dimethicone), phenyl methicone, and phenyl trimethicone. Specifically, examples thereof include KF-50-100cs (dynamic viscosity at 25°C: 100 $mm^2/s$), KF-53 (dynamic viscosity at 25°C: 175 $mm^2/s$), and KF-56 (dynamic viscosity at 25°C: 14 $mm^2/s$) (all being available from Shin-Etsu Chemical Co., Ltd.); and SH 556 FLUID (dynamic viscosity at 25°C: 14 $mm^2/s$) (available from Dow Corning Toray Co., Ltd.).

Examples of the dimethyl silicone include dimethyl polysiloxane (dimethicone). Specifically, examples thereof include KF-96A-200cs (dynamic viscosity at 25°C: 200 $mm^2/s$), KF-96A-100cs (dynamic viscosity at 25°C: 100 $mm^2/s$), KF-96A-50cs (dynamic viscosity at 25°C: 50 $mm^2/s$), KF-96A-30cs (dynamic viscosity at 25°C: 30 $mm^2/s$), KF-96A-20cs (dynamic viscosity at 25°C: 20 $mm^2/s$), KF-96A-10cs (dynamic viscosity at 25°C: 10 $mm^2/s$), KF-96A-6cs (dynamic viscosity at 25°C: 6 $mm^2/s$), KF-96A-5cs (dynamic viscosity at 25°C: 5 $mm^2/s$), KF-96L-2cs (dynamic viscosity at 25°C: 2 $mm^2/s$), KF-96L-1.5cs (dynamic viscosity at 25°C: 1.5 $mm^2/s$), and KF-96L-1cs (dynamic viscosity at 25°C: 1 $mm^2/s$) (all being available from Shin-Etsu Chemical Co., Ltd.); and SH200 C Fluid 200cs (dynamic viscosity at 25°C: 200 $mm^2/s$), SH200 C Fluid 100cs (dynamic viscosity at 25°C: 100 $mm^2/s$), SH200 C Fluid 50cs (dynamic viscosity at 25°C: 50 $mm^2/s$), SH200 C Fluid 30cs (dynamic viscosity at 25°C: 30 $mm^2/s$), SH200 C Fluid 20cs (dynamic viscosity at 25°C: 20 $mm^2/s$), SH200 C Fluid lOcs (dynamic viscosity at 25°C: 10 $mm^2/s$), SH200 C Fluid 6cs (dynamic viscosity at 25°C: 6 $mm^2/s$), SH200 C Fluid 5cs (dynamic viscosity at 25°C: 5 $mm^2/s$), SH200 C Fluid 2CS (dynamic viscosity at 25°C: 2 $mm^2/s$), SH200 C Fluid 1.5cs (dynamic viscosity at 25°C: 1.5 $mm^2/s$), and SH200 C Fluid 1cs (dynamic viscosity at 25°C: 1 $mm^2/s$) (all being available from Dow Corning Toray Co., Ltd.).

From the viewpoint of improving dispersibility of the component (B) and the component (B)' and the viewpoint that in the case where the hair cosmetic preparation is a temporary hair dye, a feeling on the hair after hair dyeing is improved, the dynamic viscosity at 25°C of the silicone oil is preferably 200 $mm^2/s$ or less, more preferably 150 $mm^2/s$ or less, and still more preferably 100 $mm^2/s$ or less. The dynamic viscosity can be measured at 25°C with an Ubbelohde viscometer on the basis of JIS Z8803: 2011 "Methods for viscosity measurement of liquid".

The silicone oil can be used alone or in combination of two or more thereof.

From the viewpoint of improving dispersibility of the component (B) and the component (B)' and the viewpoint that in the case where the hair cosmetic preparation is a temporary hair dye, a feeling on the hair after hair dyeing is improved, the content of the silicone oil in the hair cosmetic preparation is preferably 0.1% by mass or more, more preferably 0.5% by mass or more, and still more preferably 1% by mass or more, and preferably 20% by mass or less, more preferably 15% by mass or less, and still more preferably 10% by mass or less. A specific range of the content of the silicone oil in the hair cosmetic preparation is preferably 0.1 to 20% by mass, more preferably 0.5 to 15% by mass, and still more preferably 1 to 10% by mass.

<Other Component>

To the hair cosmetic preparation, a component which is typically used for the cosmetic preparation can be added in addition to the aforementioned components. Examples of the foregoing component include an alkaline agent; a surfactant; an oil, such as oils and fats and a sparingly volatile hydrocarbon; a thickener; a propellant, such as LPG (liquefied petroleum gas), pentane, dimethyl ether, and diethyl ether; and others, such as a perfume, an antiseptic, an ultraviolet absorber, a chelating agent, an antioxidant, and a vegetable extract.

A production method of the hair cosmetic preparation is not particularly limited, and the hair cosmetic preparation can be produced by stirring and mixing the aforementioned respective components by using a known device.

<Product Form and Use Method>

The cosmetic for hair of the present invention is more suitable as a hair dye cosmetic article of a mascara type, especially as a temporary hair dye cosmetic article of a mascara type.
The cosmetic for hair of the present invention is preferably used by shaking the lidded container by a hand, etc. to uniformly redisperse the liquid hair cosmetic preparation L and then taking out the applicator 2 from the container 1.
The user takes out the applicator 2 from the container 1, grasps the container lid part 5 of the applicator 2, and then pushes the application body 4 against the hair surface and moves, thereby the liquid hair cosmetic preparation L impregnated in the application body 4 can be applied to the hair. Specifically, when the user selects a hair bundle to which the liquid hair cosmetic preparation L is applied, holds and fixes a part of the hair bundle by fingers or a clip, etc. which has conventionally been used for hair care, grasps the container lid part 5 of the applicator 2 while directing toward the root to the tip of the hair, and pushes the applicator body 4 against the hair surface and moves, thereby the liquid hair cosmetic preparation L impregnated in the application body 4 can be applied to the hair.
In the case where the application body 4 is held rotatably in the peripheral direction of the application shaft 3, when the applicator 2 is moved to the direction approximately orthogonal to the shaft direction of the application shaft 3, the hair cosmetic preparation can be applied while rotating the application body 4 to the peripheral direction of the application shaft 3, and hence, such is preferred from the standpoint that liquid outage is hardly caused on the way of application, and the application can be evenly achieved in a single operation.
With respect to the aforementioned embodiments, the present invention discloses the following cosmetic for hair and a method for applying a liquid hair cosmetic preparation to the hair by an applicator.

<1> A cosmetic for hair including

a container having a liquid hair cosmetic preparation accommodated in the inside thereof; and
an applicator provided with not only an application body at one end of an application shaft but also a container lid part at the other end of the application shaft, wherein
the hair cosmetic preparation has a viscosity at 25°C of 5,000 mPa·s or less, and
the application body is provided with an absorptive member having a density of 15 kg/m$^3$ or more, preferably 20 kg/m$^3$ or more, more preferably 22 kg/m$^3$ or more, and still more preferably 25 kg/m$^3$ or more, and 95 kg/m$^3$ or less, preferably 93 kg/m$^3$ or less, and more preferably 90 kg/m$^3$ or less, and the absorptive member covers the entirety of the one end of the application shaft.

<2> A cosmetic for hair including

a container having a liquid hair cosmetic preparation accommodated in the inside thereof; and
an applicator provided with not only an application body at one end of an application shaft but also a container lid part at the other end of the application shaft, wherein
the hair cosmetic preparation has a viscosity at 25°C of 5,000 mPa·s or less, and
the application body is composed of an absorptive member having a density of 15 kg/m$^3$ or more, preferably 20 kg/m$^3$ or more, more preferably 22 kg/m$^3$ or more, and still more preferably 25 kg/m$^3$ or more, and 95 kg/m$^3$ or less, preferably 93 kg/m$^3$ or less, and more preferably 90 kg/m$^3$ or less, and the application body covers the entirety of the one end of the application shaft.

<3> The cosmetic for hair as set forth in <1> or <2>, wherein the viscosity at 25°C of the hair cosmetic preparation is more than 0 mPa·s, preferably 1 mPa·s or more, more preferably 5 mPa·s or more, and still more preferably 10 mPa·s or more, and preferably 3,000 mPa·s or less, more preferably 2,000 mPa·s or less, still more preferably 1,500 mPa·s or less, yet still more preferably 1,000 mPa·s or less, even yet still more preferably 800 mPa·s or less, even still more preferably 500 mPa·s or less, even still further preferably 300 mPa·s or less, and even yet still more further preferably 150 mPa·s or less.
<4> The cosmetic for hair as set forth in any one of <1> to <3>, wherein the hair cosmetic preparation contains the following components (A) to (C):

(A) Colorant
(B) Film-forming polymer having a silicone structure
(C) Nonaqueous solvent

<5> The cosmetic for hair as set forth in any one of <1> to <4>, wherein in the hair cosmetic preparation, the content of the component (A) is 2.0% by mass or more and 12.0% by mass or less, the content of the component (B) is 1%

by mass or more and 30% by mass or less, and the content of the component (C) is 30% by mass or more and 97% by mass or less.

<6> A cosmetic for hair including

a container having a liquid hair cosmetic preparation accommodated in the inside thereof; and
an applicator provided with not only an application body at one end of an application shaft but also a container lid part at the other end of the application shaft, wherein
the hair cosmetic preparation has a viscosity at 25°C of 1 mPa·s or more and 2,000 mPa·s or less and contains the following components (A) to (C):

(A) Colorant: 2.0% by mass or more and 12.0% by mass or less
(B) Film-forming polymer having a silicone structure: 1% by mass or more and 30% by mass or less
(C) Nonaqueous solvent: 30% by mass or more and 97% by mass or less
the application body is provided with an absorptive member having a density of 15 kg/$m^3$ or more and 95 kg/$m^3$ or less, and the absorptive member covers the entirety of the one end of the application shaft.

<7> A cosmetic for hair including

a container having a liquid hair cosmetic preparation accommodated in the inside thereof; and
an applicator provided with not only an application body at one end of an application shaft but also a container lid part at the other end of the application shaft, wherein
the hair cosmetic preparation has a viscosity at 25°C of 5 mPa·s or more and 1,500 mPa·s or less and contains the following components (A) to (C):

(A) Colorant: 2.0% by mass or more and 12.0% by mass or less
(B) Film-forming polymer having a silicone structure: 1% by mass or more and 30% by mass or less
(C) Nonaqueous solvent: 30% by mass or more and 97% by mass or less
the application body is provided with an absorptive member having a density of 15 kg/$m^3$ or more and 95 kg/$m^3$ or less, an outer diameter of 5 to 35 mm, a thickness of 0.5 to 8 mm, and a length in the application shaft direction of 10 to 60 mm, and the absorptive member covers the entirety of the one end of the application shaft.

<8> A cosmetic for hair including

a container having a liquid hair cosmetic preparation accommodated in the inside thereof; and
an applicator provided with not only an application body at one end of an application shaft but also a container lid part at the other end of the application shaft, wherein
the hair cosmetic preparation has a viscosity at 25°C of 5 mPa·s or more and 1,500 mPa·s or less and contains the following components (A) to (C):

(A) Colorant: 2.0% by mass or more and 12.0% by mass or less
(B) Film-forming polymer having a silicone structure: 1% by mass or more and 30% by mass or less
(C) Nonaqueous solvent: 30% by mass or more and 97% by mass or less
the application body is provided with an absorptive member having a density of 15 kg/$m^3$ or more and 95 kg/$m^3$ or less, an outer diameter of 8 to 25 mm, a thickness of 1 to 6 mm, and a length in the application shaft direction of 15 to 50 mm, and the absorptive member covers the entirety of the one end of the application shaft.

<9> A cosmetic for hair including

a container having a liquid hair cosmetic preparation accommodated in the inside thereof; and
an applicator provided with not only an application body at one end of an application shaft but also a container lid part at the other end of the application shaft, wherein
the hair cosmetic preparation has a viscosity at 25°C of 5 mPa·s or more and 1,500 mPa·s or less and contains the following components (A) to (C):

(A) Colorant: 2.0% by mass or more and 12.0% by mass or less
(B) Film-forming polymer having a silicone structure: 1% by mass or more and 30% by mass or less

(C) Nonaqueous solvent: 30% by mass or more and 97% by mass or less
the application body is provided with an absorptive member having a density of 15 $kg/m^3$ or more and 95 $kg/m^3$ or less, an outer diameter of 10 to 15 mm, a thickness of 1.5 to 4 mm, and a length in the application shaft direction of 20 to 40 mm, and the absorptive member covers the entirety of the one end of the application shaft.

<10> A cosmetic for hair including

a container having a liquid hair cosmetic preparation accommodated in the inside thereof; and
an applicator provided with not only an application body at one end of an application shaft but also a container lid part at the other end of the application shaft, wherein
the hair cosmetic preparation has a viscosity at 25°C of 10 mPa·s or more and 300 mPa·s or less and contains the following components (A) to (C):

(A) Colorant: 2.0% by mass or more and 12.0% by mass or less
(B) Film-forming polymer having a silicone structure: 1% by mass or more and 30% by mass or less
(C) Nonaqueous solvent: 30% by mass or more and 97% by mass or less
the application body is provided with an absorptive member having a density of 15 $kg/m^3$ or more and 95 $kg/m^3$ or less, and the absorptive member covers the entirety of the one end of the application shaft.

<11> A cosmetic for hair including

a container having a liquid hair cosmetic preparation accommodated in the inside thereof; and
an applicator provided with not only an application body at one end of an application shaft but also a container lid part at the other end of the application shaft, wherein
the hair cosmetic preparation has a viscosity at 25°C of 10 mPa·s or more and 300 mPa·s or less and contains the following components (A) to (C):

(A) Colorant: 2.0% by mass or more and 12.0% by mass or less
(B) Film-forming polymer having a silicone structure: 1% by mass or more and 30% by mass or less
(C) Nonaqueous solvent: 30% by mass or more and 97% by mass or less
the application body is provided with an absorptive member having a density of 15 $kg/m^3$ or more and 95 $kg/m^3$ or less, an outer diameter of 10 to 15 mm, a thickness of 1.5 to 4 mm, and a length in the application shaft direction of 20 to 40 mm, and the absorptive member covers the entirety of the one end of the application shaft.

<12> The cosmetic for hair as set forth in any one of <1> to <11>, wherein a coverage ratio of the application body by the absorptive member is 50% or more, preferably 70% or more, more preferably 90% or more, and still more preferably 100% of the outer surface of the application body.
<13> The cosmetic for hair as set forth in any one of <1> to <12>, wherein the application body is one composed of the absorptive member, and the application body covers the entirety of the one end of the application shaft.
<14> The cosmetic for hair as set forth in any one of <1> to <13>, wherein in the applicator, the application body is held rotatably in the peripheral direction of the application shaft.
<15> The cosmetic for hair as set forth in any one of <1> to <14>, wherein the material of the absorptive member is polyurethane.
<16> The cosmetic for hair as set forth in any one of <1> to <15>, wherein the application body is provided with an approximately cylindrical absorptive member covering the entirety of the one end of the application shift.
<17> The cosmetic for hair as set forth in any one of <1> to <15>, wherein the application body is composed of an approximately cylindrical absorptive member covering the entirety of the one end of the application shift.
<18> The cosmetic for hair as set forth in any one of <1> to <17>, wherein the container is provided with a wiping member within a neck part thereof.
<19> The cosmetic for hair as set forth in any one of <3> to <18>, wherein the component (A) preferably contains an interference pearl pigment (A1) and a colorant (A2) other than the interference pearl pigment.
<20> The cosmetic for hair as set forth in <19>, wherein the content of the component (A1) in the hair cosmetic preparation is preferably 1.5% by mass or more, more preferably 2.5% by mass or more, still more preferably 3.0% by mass or more, yet still more preferably 3.5% by mass or more, and even yet still more preferably 4.0% by mass or more, and preferably 10% by mass or less, more preferably 9.5% by mass or less, still more preferably 9.0% by mass or less, and yet still more preferably 8.5% by mass or less.

<21> The cosmetic for hair as set forth in <19> or <20>, wherein the content of the component (A2) in the hair cosmetic preparation is preferably 0.5% by mass or more, more preferably 0.8% by mass or more, and still more preferably 1.0% by mass or more, and preferably 8.0% by mass or less, more preferably 7.0% by mass or less, still more preferably 6.0% by mass or less, yet still more preferably 5.0% by mass or less, and even yet still more preferably 4.0% by mass or less.

<22> The cosmetic for hair as set forth in any one of <3> to <21>, wherein the content of the component (A) in the hair cosmetic preparation is preferably 3.0% by mass or more, more preferably 3.5% by mass or more, still more preferably 4.0% by mass or more, yet still more preferably 5.0% by mass or more, and even yet still more preferably 6.0% by mass or more, and preferably 11.0% by mass or less, more preferably 10.0% by mass or less, and still more preferably 9.5% by mass or less.

<23> The cosmetic for hair as set forth in any one of <19> to <22>, wherein a mass ratio [(A1)/{(A1) + (A2)}] of the component (A1) to the total content of the component (A1) and the compound (A2) is preferably 0.30 or more, more preferably 0.40 or more, still more preferably 0.50 or more, and yet still more preferably 0.60 or more, and preferably 0.96 or less, more preferably 0.94 or less, still more preferably 0.92 or less, and yet still more preferably 0.90 or less.

<24> The cosmetic for hair as set forth in any one of <4> to <23>, wherein the component (B) is preferably a poly(N-acylalkyleneimine)-modified organopolysiloxane, more preferably an organopolysiloxane in which a poly(N-acylalkyleneimine) segment composed of a repeating unit represented by the following general formula (1) is bound to at least two silicon atoms of an organopolysiloxane segment constituting the main chain via a hetero atom-containing alkylene group, still more preferably one or more selected from the group consisting of a poly(N-formylethyleneimine)organosiloxane, a poly(N-acetylethyleneimine)organosiloxane, and a poly(N-propionylethyleneimine)organosiloxane, yet still more preferably a poly(N-propionylethyleneimine)organosiloxane, and even yet still more preferably poly(N-propionylethyleneimine)methylsiloxane:

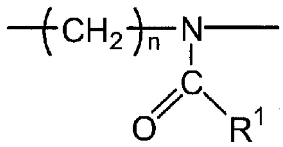

(1)

wherein $R^1$ represents a hydrogen atom, an alkyl group having 1 or more and 22 or less carbon atoms, an aralkyl group having 7 or more and 22 or less carbon atoms, or an aryl group having 6 or more and 22 or less carbon atoms; and n represents 2 or 3.

<25> The cosmetic for hair as set forth in any one of <4> to <24>, wherein the content of the component (B) in the hair cosmetic preparation is preferably 2% by mass or more, more preferably 3% by mass or more, and still more preferably 4% by mass or more, and preferably 20% by mass or less, more preferably 15% by mass or less, and still more preferably 12% by mass or less.

<26> The cosmetic for hair as set forth in any one of <4> to <25>, wherein a mass ratio [(B)/(A)] of the component (B) to the component (A) in the hair cosmetic preparation is preferably 0.2 or more, more preferably 0.5 or more, and still more preferably 0.8 or more, and preferably 3 or less, more preferably 1.4 or less, and still more preferably 1.2 or less.

<27> The cosmetic for hair as set forth in any one of <4> to <26>, wherein the hair cosmetic preparation further contains, as a component (B)', a polymer other than the component (B), and preferably a cationic polymer.

<28> The cosmetic for hair as set forth in <27>, wherein a cation charge density of the cationic polymer is preferably 0.05 meq/g or more, more preferably 0.1 meq/g or more, still more preferably 0.2 meq/g or more, yet still more preferably 0.4 meq/g or more, and even yet still more preferably 0.5 meq/g or more, and preferably 6.5 meq/g or less, more preferably 3.8 meq/g or less, still more preferably 2.5 meq/g or less, yet still more preferably 2.0 meq/g or less, and even yet still more preferably 1.5 meq/g or less.

<29> The cosmetic for hair as set forth in <27> or <28>, wherein the cationic polymer is at least one selected from the group consisting of a cationized guar gum, a cationized tara gum, a cationized locust bean gum, a cationized polyvinyl alcohol, a vinylpyrrolidone/N,N-dimethylaminoethyl methacrylate copolymer diethyl sulfate, a vinylpyrrolidone/dimethylaminopropyl methacrylamide/lauryl dimethylaminopropyl methacrylamide copolymer, polydimethylmethylenepiperidinium chloride, a dimethyldiallylammonium chloride/acrylamide copolymer, a vinylpyrrolidone/N,N-dimethylaminoethyl methacrylate copolymer diethyl sulfate, a vinylpyrrolidone/N,N-dimethylaminoethyl methacrylate copolymer, an N,N-dimethylaminoethyl methacrylate diethyl sulfate/N,N-dimethyl acrylamide/polyethylene glycol dimethacrylate copolymer, ammonium-modified hydroxyethyl cellulose, and an acrylamide/DMAPA acrylate/methoxy methacrylate PEG copolymer; preferably at least one selected from the group consisting of a dimethyldiallylammonium chloride/acrylamide copolymer, a vinylpyrrolidone/N,N-dimethylaminoethyl methacrylate copolymer diethyl sulfate, an N,N-dimethylaminoethyl methacrylate diethyl sulfate/N,N-dimethyl acrylamide/polyethylene glycol

dimethacrylate copolymer, ammonium-modified hydroxyethyl cellulose, and an acrylamide/DMAPA acrylate/methoxy methacrylate PEG copolymer; more preferably at least one selected from the group consisting of a dimethyldiallylammonium chloride/acrylamide copolymer, a vinylpyrrolidone/N,N-dimethylaminoethyl methacrylate copolymer diethyl sulfate, and an N,N-dimethylaminoethyl methacrylate diethyl sulfate/N,N-dimethyl acrylamide/polyethylene glycol dimethacrylate copolymer; and still more preferably an N,N-dimethylaminoethyl methacrylate diethyl sulfate/N,N-dimethyl acrylamide/polyethylene glycol dimethacrylate copolymer.

<30> The cosmetic for hair as set forth in any one of <27> to <29>, wherein the content of the component (B)' in the hair cosmetic preparation is preferably 0.05% by mass or more, more preferably 0.1% by mass or more, and still more preferably 0.2% by mass or more, and preferably 5% by mass or less, more preferably 3% by mass or less, still more preferably 2.5% by mass or less, and yet still more preferably 1% by mass or less.

<31> The cosmetic for hair as set forth in any one of <4> to <30>, wherein the content of ethanol in the component (C) is preferably 30% by mass or more, more preferably 50% by mass or more, and still more preferably 80% by mass or more.

<32> The cosmetic for hair as set forth in any one of <4> to <31>, wherein the content of the component (C) in the hair cosmetic preparation is preferably 50% by mass or more, and more preferably 70% by mass or more, and preferably 97% by mass or less.

<33> The cosmetic for hair as set forth in any one of <1> to <32>, wherein the content of water in the hair cosmetic preparation is preferably 40% by mass or less, more preferably 30% by mass or less, still more preferably 20% by mass or less, yet still more preferably 15% by mass or less, even yet still more preferably 10% by mass or less, even yet still more preferably 5% by mass or less, even still more preferably 2% by mass or less, and even still more further preferably 1% by mass or less.

<34> The cosmetic for hair as set forth in any one of <1> to <33>, wherein the hair cosmetic preparation further contains a silicone oil in an amount of preferably 0.1% by mass or more, more preferably 0.5% by mass or more, and still more preferably 1% by mass or more, and preferably 20% by mass or less, more preferably 15% by mass or less, and still more preferably 10% by mass or less.

<35> The cosmetic for hair as set forth in any one of <1> to <34>, which is a hair dye cosmetic article of a mascara type, and preferably a temporary hair dye cosmetic article of a mascara type.

<36> A method for applying a liquid hair cosmetic preparation with the applicator to the hair by using the cosmetic for hair as set forth in any one of <1> to <35>, including

a step of selecting a hair bundle to which the liquid hair cosmetic preparation is applied by a user;
a step of holding and fixing the hair bundle by fingers or a clip by the user; and
a step of pushing the application body of the applicator against the hair surface and moving it.

## Examples

The present invention is hereunder described by reference to Examples, but it should be construed that the present invention is not limited to the scope of the Examples.

### (Viscosity Measurement)

With respect to the viscosity of the hair cosmetic preparation (temporary hair dye), 70 to 80 g of the hair cosmetic preparation was charged in a screw tube No. 8 (available from Maruemu Corporation, capacity: 110 mL) and shaken by hand 100 times in a width of about 30 cm for 30 seconds such that it became uniform; and further, immediately after standing up 30 times at a speed of one time per second, using a B-type viscosity (TV-10M, available from Toki Sangyo Co., Ltd.), the viscosity was measured at a temperature of 25±1°C for 1 minute under conditions of using a rotor No. M1 for up to viscosities of 100 mPa·s at a rotation speed of 100 rpm, using a rotor No. M2 in a viscosity range of 100 to 500 mPa·s at a rotation speed of 60 rpm, using a rotor No. M3 in a viscosity range of 500 to 2,000 mPa·s at a rotation speed of 60 rpm, and using a rotor No. M4 in a viscosity range of 2,000 to 10,000 mPa·s at a rotation speed of 60 rpm.

### (Preparation of Hair Cosmetic Preparation)

A mixture of the component (A1) and the component (A2) as shown in Table 1 was prepared and then added to ethanol that is the component (C), and the contents were mixed until they became uniform. To this, the remaining component(s) was added, and the contents were mixed until they became uniform, to obtain a hair cosmetic preparation (temporary hair dye) as shown in Table 1.

(Fabrication of Applicator)

Using an application body composed of an absorptive member (polyurethane-made sponge) as shown in Table 2, a test applicator 21 having a shape shown in Figs. 6 and 7 and a test applicator 22 for comparison having a shape shown in Figs. 8 and 9 were fabricated. The shape of the applicator provided with a cylindrical application body is the test applicator 21, and the shape of the applicator provided with a platy application body is the test applicator 22 for comparison.

Fig. 6 is a side view of the test applicator 21 according to the present embodiment, and Fig. 7 is a perspective view thereof. In Figs. 6 and 7, 31 is an application shaft, and 41 is an application body. The material of other portion than the application body of the test applicator 21 is polypropylene, the application shaft 31 is in a columnar form, and the application body is in a cylindrical form and held rotatably in the peripheral direction of the application shaft. The application body in Fig. 6 was set so as to have a length h1 in the application shaft direction of 30 mm, an outer diameter d1 of 12 mm, and an inner diameter d2 of 5.5 mm.

Fig. 8 is a side view of the test applicator 22 for comparison as seen from an application body-covered surface of an application shaft, and Fig. 9 is a perspective view thereof. In Figs. 8 and 9, 32 is an application shaft, and 42 is an application body. The material of other portion than the application body of the test applicator 22 for comparison is polypropylene, the application shaft 32 is in a square pillar form, and the application body 42 is in a platy form, is fixed at one end of the application shaft 32, and covers only a part of the side face at one end of the application shaft. In addition, the application body 42 in Figs. 8 and 9 was set so as to have a length h2 in the application shaft direction of 30 mm, a width w of 12 mm, and a thickness t of 2.5 mm.

(Preparation of Tress for Evaluation)

A black hair tress having a length of 30 cm and a mass of 10 g (BS-B3A, manufactured by Beaulax Co., Ltd.) was prepared. This black tress was washed once with a plain hair shampoo having the following composition, air-dried, and then used for the following evaluations.

[Composition of Plain Shampoo]

| Component | (% by mass) |
|---|---|
| Sodium polyoxyethylene(2) lauryl ether sulfate (*1) | 15.5 |
| Lauric acid diethanolamide (*2) | 1.5 |
| Tetrasodium edetate | 0.3 |
| Sodium benzoate | 1.43 |
| Purified water | Balance |
| Total | 100.0 |

*1: 42.0% by mass as EMAL 227 (available from Kao Corporation, active ingredient: 27% by mass)
*2: AMINON L-02 (available from Kao Corporation)

Examples 1 to 15 and Comparative Examples 1 to 16 (Evaluation of Cosmetic for hair)

Using cosmetic for hairs each composed of a combination of a hair cosmetic preparation and a test applicator provided with an application body shown in Tables 3 and 4, various evaluations were performed by the following methods.

<Impregnation Method of Hair Cosmetic Preparation into Application Body>

70 g of the liquid hair cosmetic preparation was filled in a screw tube (No. 8) and thoroughly shaken upward and downward in a lidded state, thereby uniformly dispersing the colorant. Into this hair cosmetic preparation, the applicator was inserted from the application body side, and after keeping for 10 seconds in a state where the whole of the application body was completely dipped in the hair cosmetic preparation, the application body was lifted up from the hair cosmetic preparation. At this time, as shown in Figs. 7 and 9, the application body was drawn up in the arrow direction from the other side of the application body of the application shaft through a wiping hole of a wiping member such that the application body passed through the wiping hole, thereby removing the excessive hair cosmetic preparation of the application body surface. In Fig. 7, 81 is concerned with a perspective view of the wiping member used for the

test applicator 21, and 81c is a wiping hole. In addition, in Fig. 9, 82 is concerned with a perspective view of the wiping member used for the test applicator 22 for comparison, and 82c is a wiping hole.

A shape of the wiping hole is a similar figure to a cross-sectional shape of the application body and the application shaft of the applicator the direction perpendicular to in the shaft direction, and an area of the wiping hole is 1.36 times the cross-sectional area of the cross section thereof. In addition, a thickness of the wiping member in the application shaft direction is 2 mm.

<Liquid Holding Amount>

A weight of the applicator before impregnating the hair cosmetic preparation and a weight of the applicator after impregnating the hair cosmetic preparation in the application body by the aforementioned method were measured, and a liquid amount (liquid holding amount) held by the application body was calculated from a difference in weight before and after impregnation of the hair cosmetic preparation. The liquid holding amount (g) was rounded off from the third decimal place and shown in Tables 3 and 4.

<Goodness of Liquid Sustainability>

After impregnating the hair cosmetic preparation in the application body of the applicator by the aforementioned method, the applicator was inclined such that the application shaft direction became horizontal and allowed to stand for 15 minutes, and the presence or absence of dripping of the hair cosmetic preparation was visually observed. Furthermore, the presence or absence of liquid dripping on the occasion of applying to the aforementioned tress for evaluation (dripping from the tress or dripping from the applicator) was visually observed by five evaluators and evaluated according to the following evaluation criteria.

{Evaluation Criteria and Score}

1: The liquid dripping from the applicator having been allowed to incline and stand after liquid holding was not seen, and also the liquid dripping was not seen during the application operation of all evaluators.
2: The liquid dripping from the applicator having been allowed to incline and stand after liquid holding was not seen, but the liquid dripping was seen during the application operation of some of the evaluators (tolerance level from practical use).
3: The liquid dripping was seen from the applicator having been allowed to incline and stand after liquid holding.

<Length of Dyeing by One Coat>

After the impregnating the hair cosmetic preparation in the application body of the applicator by the aforementioned method, it was applied to the aforementioned tress for evaluation from the root to the tip, and the length of dyeing by one coat was measured. The length (cm) of dyeing by one coat was rounded off from the first decimal place and shown in Tables 3 and 4.

<No Uneven Dyeing by One Coat>

After the impregnating the hair cosmetic preparation in the application body of the applicator by the aforementioned method, it was applied to the aforementioned tress for evaluation from the root to the tip by one coat, followed by air-drying. With respect to the applied tress, the presence or absence of uneven dyeing was visually observed by five panelists and evaluated according to the following evaluation criteria. Scores according to the following evaluation criteria are shown in Tables 3 and 4.

[Evaluation Criteria]

a: Uniformly finished
b: Slight uneven dyeing (though an aggregate of the colorant is not found, dyeing is partly non-uniform)
c: Evident uneven dyeing (an aggregate of the colorant is confirmed)

[Evaluation Criteria and Score]

1: No person gave the evaluation "c", and two or more persons gave the evaluation "a".
2: Not more than one person gave the evaluation "c", and not more than one person gave the evaluation "a".
3: Two or more persons gave the evaluation "c".

Table 1

| % by mass | | Hair cosmetic preparation | | | | |
|---|---|---|---|---|---|---|
| | | A | B | C | D | E |
| (A1) | Micaceous titanium A *1 | 6.5 | 6.5 | 6.5 | 6.5 | 6.5 |
| (A2) | Yellow No. 401 /barium sulfate *2 | 0.63 | 0.63 | 0.63 | 0.63 | 0.63 |
| | Red No. 226 *3 | 0.95 | 0.95 | 0.95 | 0.95 | 0.95 |
| | Blue No. 404 *4 | 0.95 | 0.95 | 0.95 | 0.95 | 0.95 |
| (B) | Polysilicone-9 *5 | 9.0 | 9.0 | 9.0 | 9.0 | 9.0 |
| (B)' | Polyquaternium-52 *6 | 0 | 0.3 | 0.9 | 2.3 | 3.2 |
| (C) | Ethanol | 76.96 | 76.66 | 76.06 | 74.66 | 73.76 |
| Methylphenyl polysiloxane *7 | | 5 | 5 | 5 | 5 | 5 |
| Total | | 100 | 100 | 100 | 100 | 100 |
| Content of component (A1) (% by mass) | | 6.5 | 6.5 | 6.5 | 6.5 | 6.5 |
| Content of component (A2) (% by mass) | | 2.54 | 2.54 | 2.54 | 2.54 | 2.54 |
| Total content of the components (A1) and (A2) (% by mass) | | 9.04 | 9.04 | 9.04 | 9.04 | 9.04 |
| Mass ratio [(A1)/{(A1) + (A2)}] | | 0.72 | 0.72 | 0.72 | 0.72 | 0.72 |
| Mass ratio [(B)/(A1)] | | 1.38 | 1.38 | 1.38 | 1.38 | 1.38 |
| Mass ratio [(B)/(A)] | | 1.00 | 1.00 | 1.00 | 1.00 | 1.00 |
| Viscosity (mPa·s/25°C) | | 7 | 14 | 138 | 1120 | 2670 |

The components described in Table 1 are shown below. In addition, the blending amounts of the components described in Table 1 are each an effective amount (% by mass).

*1: Timiron Super Blue (available from Merck)
*2: 70% Yellow No. 401 barium sulfate (available from Kishi Kasei Co., Ltd.)
*3: D&C RED No. 30 (available from Daito Kasei Kogyo Co., Ltd.)
*4: Blue No. 404 (color of law) (available from Kishi Kasei Co., Ltd.)
*5: OS-88E-E (available from Kao Corporation)
*6: Sofcare KG-101-E (available from Kao Corporation, cation charge density: 0.94 meq/g)
*7: Silicone SH 556 FLUID (available from Dow Corning Toray Co., Ltd.)

Table 2

| | Application body | | | | | | | |
|---|---|---|---|---|---|---|---|---|
| | a | b | c | Comparison a | Comparison b | Comparison c | Comparison d | Comparison e |
| Density of absorptive member ($kg/m^2$) | 30 | 60 | 85 | 13 | 100 | 30 | 60 | 85 |
| Shape of application body | Cylindrical | Cylindrical | Cylindrical | Cylindrical | Cylindrical | Platy | Platy | Platy |

Table 3

| | | Example | | | | | | | | | | | | | | |
|---|---|---|---|---|---|---|---|---|---|---|---|---|---|---|---|---|
| | | 1 | 2 | 3 | 4 | 5 | 6 | 7 | 8 | 9 | 10 | 11 | 12 | 13 | 14 | 15 |
| Hair cosmetic preparation | No. | A | A | A | B | B | B | C | C | C | D | D | D | E | E | E |
| | Viscosity (mPa·s/25°C) | 7 | 7 | 7 | 14 | 14 | 14 | 138 | 138 | 138 | 1120 | 1120 | 1120 | 2670 | 2670 | 2670 |
| Application body | No. | a | b | c | a | b | c | a | b | c | a | b | c | a | b | c |
| | Density of absorptive member ($kg/m^3$) | 30 | 60 | 85 | 30 | 60 | 85 | 30 | 60 | 85 | 30 | 60 | 85 | 30 | 60 | 85 |
| | Shape of application body | Cylindrical | Cylindrical | Cylindrical | Cylindrical | Cylindrical | Cylindrical | Cylindrical | Cylindrical | Cylindrical | Cylindrical | Cylindrical | Cylindrical | Cylindrical | Cylindrical | Cylindrical |
| Evaluation results | Liquid holding amount (g) | 1.62 | 1.93 | 1.86 | 1.77 | 1.85 | 1.92 | 1.52 | 1.90 | 1.88 | 1.63 | 1.79 | 1.92 | 1.35 | 2.01 | 1.90 |
| | Goodness of liquid sustainability | 2 | 2 | 2 | 1 | 1 | 1 | 1 | 1 | 1 | 1 | 1 | 1 | 1 | 1 | 1 |
| | Length of dyeing by one coat (cm) | 30 | 30 | 30 | 30 | 30 | 30 | 30 | 30 | 30 | 26 | 28 | 28 | 16 | 19 | 21 |
| | No uneven dyeing by one coat | 1 | 1 | 1 | 1 | 1 | 1 | 1 | 1 | 1 | 1 | 1 | 1 | 2 | 2 | 2 |

Table 4

| | | Comparative Example | | | | | | | | | | | | | | | |
|---|---|---|---|---|---|---|---|---|---|---|---|---|---|---|---|---|---|
| | | 1 | 2 | 3 | 4 | 5 | 6 | 7 | 8 | 9 | 10 | 11 | 12 | 13 | 14 | 15 | 16 |
| Hair cosmetic preparation | No. | A | A | B | B | B | B | B | C | C | C | C | C | D | D | E | E |
| | Viscosity (mPa·s/ 25°C) | 7 | 7 | 14 | 14 | 14 | 14 | 14 | 138 | 138 | 138 | 138 | 138 | 1120 | 1120 | 2670 | 2670 |
| Application body | No. | Comparison a | Comparison b | Comparison a | Comparison b | Comparison c | Comparison d | Comparison e | Comparison a | Comparison b | Comparison c | Comparison d | Comparison e | Comparison a | Comparison b | Comparison a | Comparison b |
| | Density of absorptive member (kg/m$^3$) | 13 | 100 | 13 | 100 | 30 | 60 | 85 | 13 | 100 | 30 | 60 | 85 | 13 | 100 | 13 | 100 |
| | Shape of application body | Cylindrical | Cylindrical | Cylindrical | Cylindrical | Platy | Platy | Platy | Cylindrical | Cylindrical | Platy | Platy | Platy | Cylindrical | Cylindrical | Cylindrical | Cylindrical |
| Evaluation results | Liquid holding amount (g) | 1.03 | 1.94 | 0.98 | 1.88 | 0.68 | 0.78 | 0.82 | 0.81 | 1.93 | 0.43 | 0.69 | 0.88 | 0.64 | 2.04 | 0.48 | 1.88 |
| | Goodness of liquid sustainability | 2 | 3 | 1 | 3 | 2 | 2 | 2 | 1 | 2 | 1 | 1 | 1 | 1 | 2 | 1 | 2 |
| | Length of dyeing by one coat (cm) | 25 | 12 | 26 | 17 | 21 | 26 | 30 | 26 | 26 | 14 | 22 | 30 | 21 | 21 | 12 | 18 |
| | No uneven dyeing by one coat | 2 | 1 | 2 | 2 | 1 | 1 | 1 | 2 | 2 | 1 | 1 | 1 | 3 | 3 | 3 | 3 |

Industrial Applicability

In accordance with the present invention, it is possible to provide a cosmetic for hair which is suitable as a cosmetic for hair of a mascara type, especially as a temporary hair dye cosmetic article and hardly causes liquid dripping or liquid outage during an application operation and can be evenly applied in a single operation to the hair from the root to the tip even against long hairs.

Reference Signs List

| | |
|---|---|
| 1: | Container |
| 1a: | Male screw of container |
| 2, 21, 22: | Applicator |
| 3, 31, 32: | Application shaft |
| 4, 41, 42: | Application body |
| 5: | Container lid part |
| 5a: | Female screw of container lid part |
| 6: | Rotation body |
| 7: | Application body holder |
| 8, 81, 82: | Wiping member |
| 8a: | Slit |
| 8b: | Disc-like wiping part |
| 8c, 81c, 82c: | Wiping hole |
| 9: | Stirring ball |
| 100: | Cosmetic for hair |

**Claims**

**1.** A cosmetic for hair comprising

a container having a liquid hair cosmetic preparation accommodated in the inside thereof; and
an applicator provided with not only an application body at one end of an application shaft but also a container lid part at the other end of the application shaft, wherein
the hair cosmetic preparation has a viscosity at 25°C of 5,000 mPa·s or less, and
the application body is provided with an absorptive member having a density of 15 $kg/m^3$ or more and 95 $kg/m^3$ or less, and the absorptive member covers the entirety of the one end of the application shaft.

**2.** A cosmetic for hair comprising

a container having a liquid hair cosmetic preparation accommodated in the inside thereof; and
an applicator provided with not only an application body at one end of an application shaft but also a container lid part at the other end of the application shaft, wherein
the hair cosmetic preparation has a viscosity at 25°C of 5,000 mPa·s or less, and
the application body is composed of an absorptive member having a density of 15 $kg/m^3$ or more and 95 $kg/m^3$ or less, and the application body covers the entirety of the one end of the application shaft.

**3.** The cosmetic for hair according to claim 1 or 2, wherein in the applicator, the application body is held rotatably in the peripheral direction of the application shaft.

**4.** The cosmetic for hair according to any one of claims 1 to 3, wherein the material of the absorptive member is polyurethane.

**5.** The cosmetic for hair according to any one of claims 1 to 4, wherein the hair cosmetic preparation contains the following components (A) to (C):

(A) Colorant

(B) Film-forming polymer having a silicone structure
(C) Nonaqueous solvent

**6.** The cosmetic for hair according to any one of claims 1 to 5, which is a temporary cosmetic for hair of a mascara type.

**7.** The cosmetic for hair according to any one of claims 1 to 6, wherein the viscosity at 25°C of the hair cosmetic preparation is 5 mPa·s or more and 1,500 mPa·s or less.

[Fig. 1]

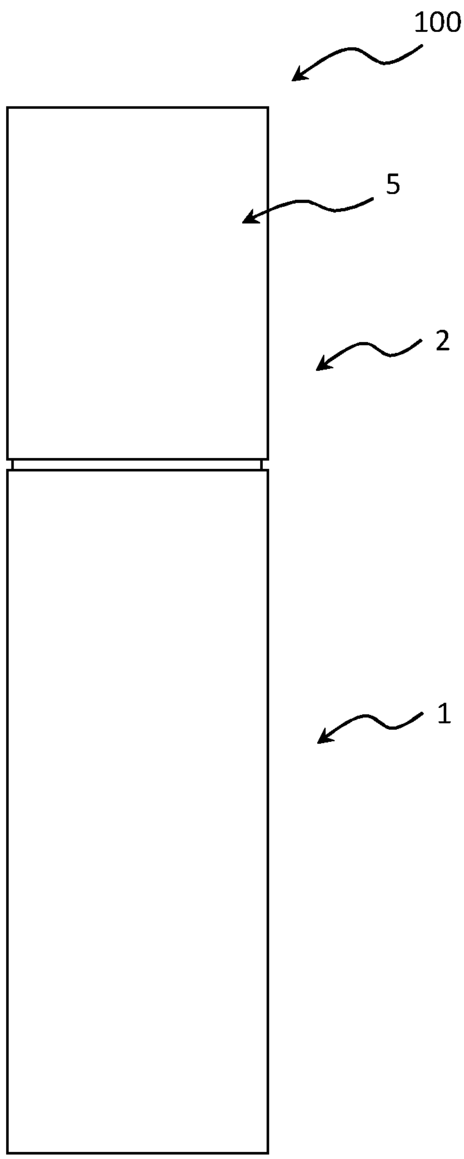

[Fig. 2]

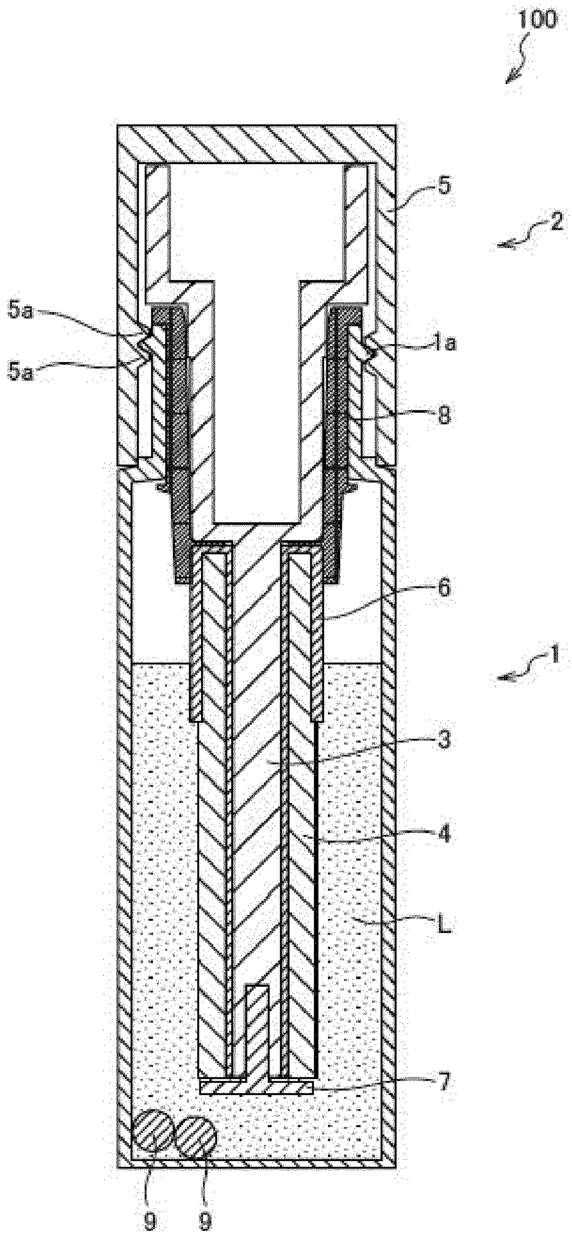

[Fig. 3]

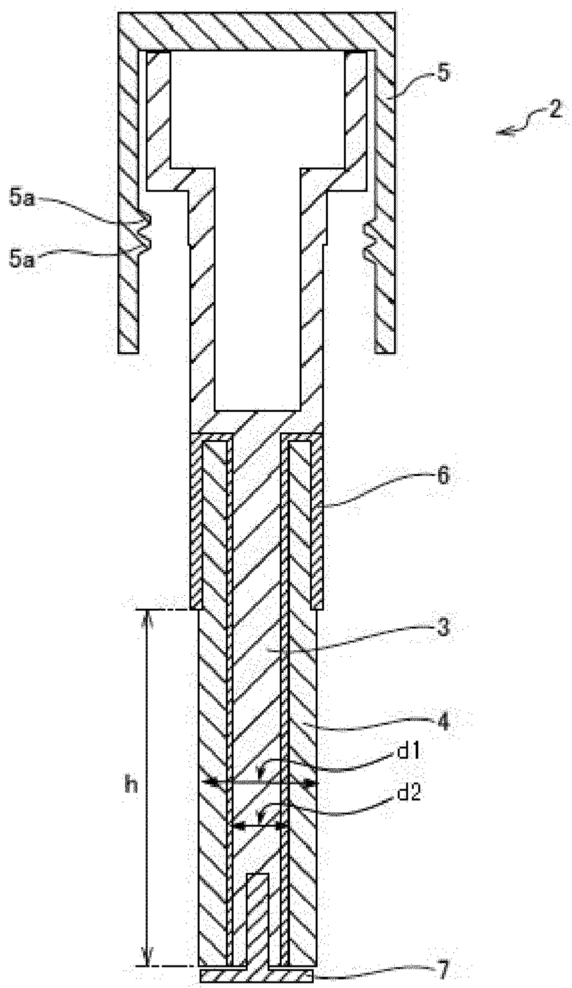

[Fig. 4]

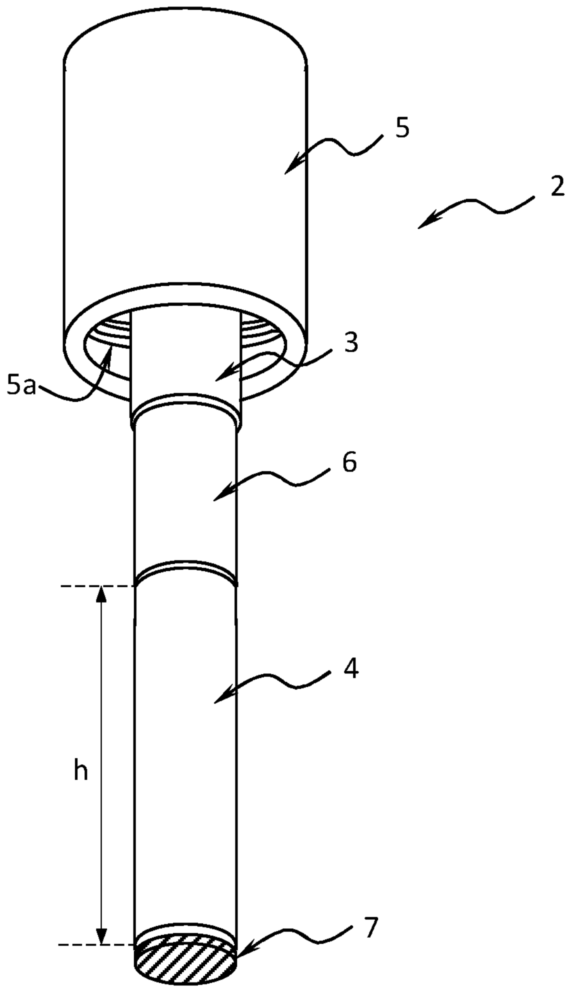

[Fig. 5]

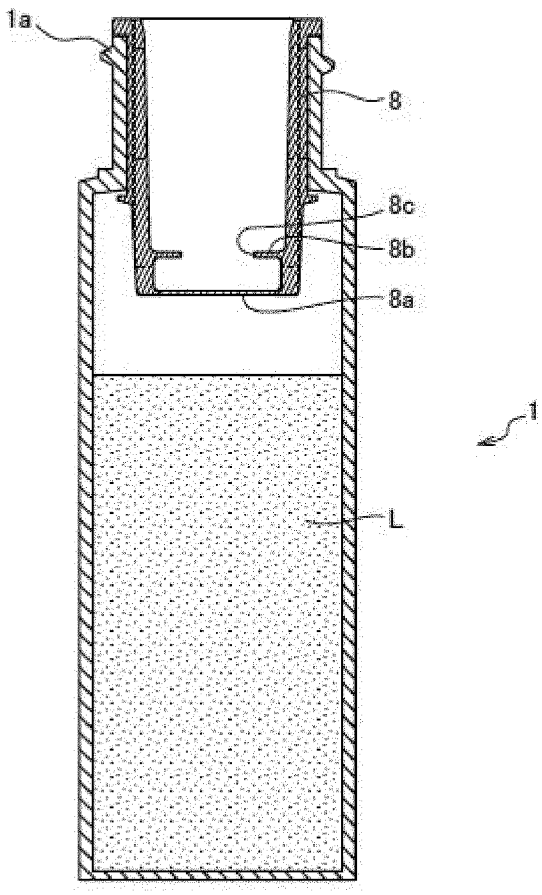

[Fig. 6]

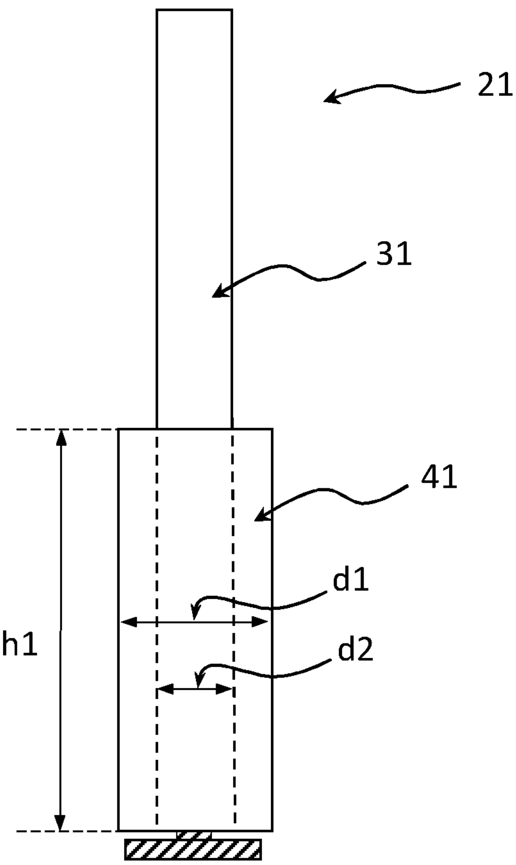

[Fig. 7]

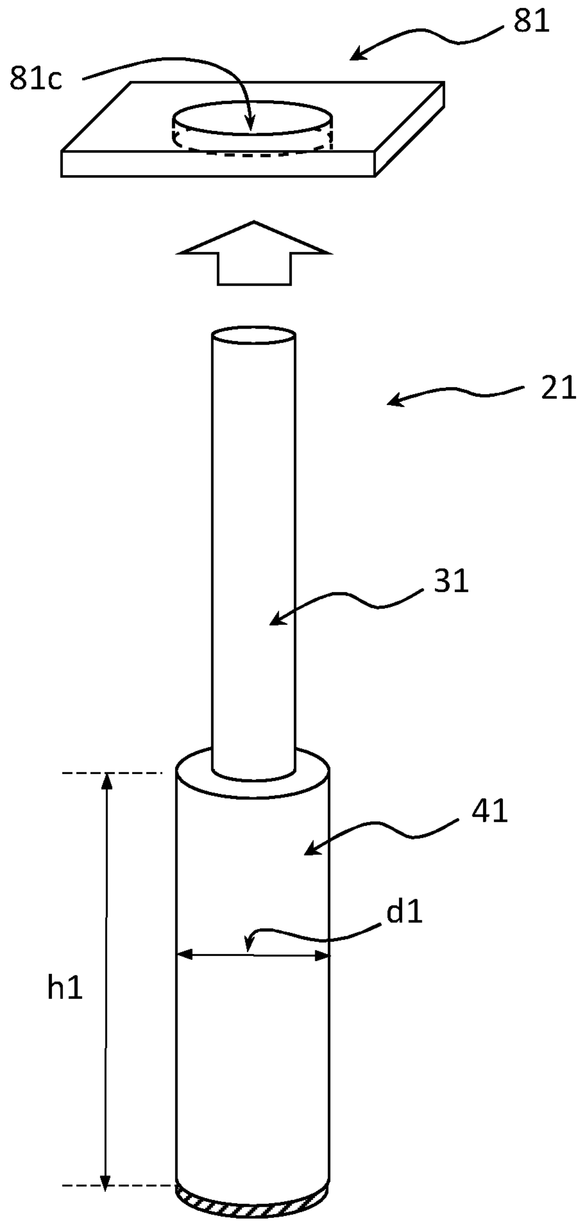

[Fig. 8]

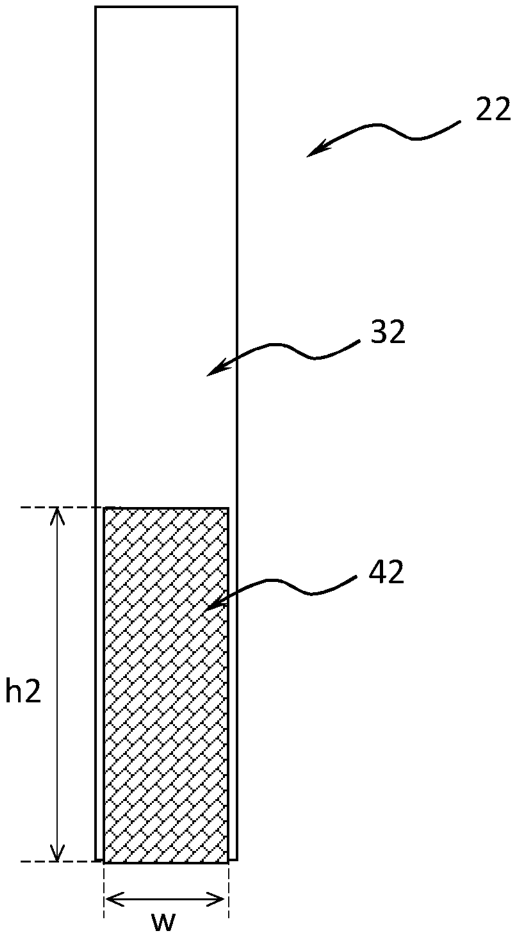

[Fig. 9]

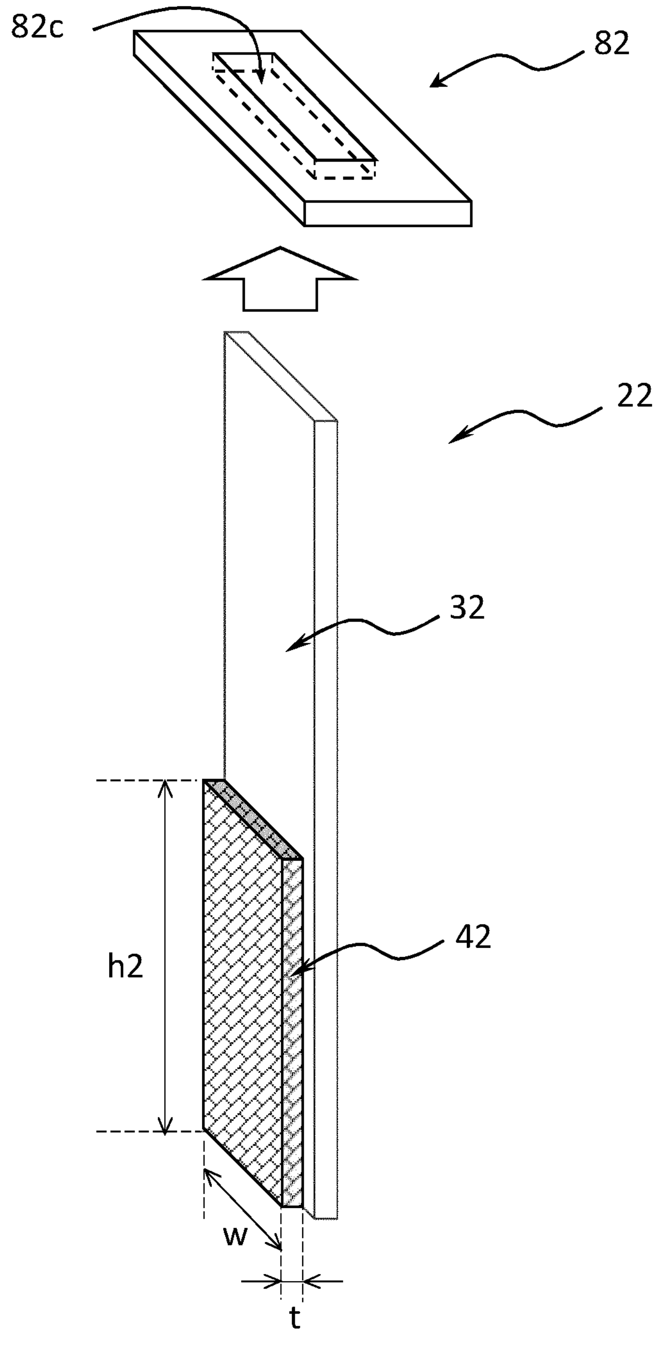

**INTERNATIONAL SEARCH REPORT**

International application No.
PCT/JP2020/015486

A. CLASSIFICATION OF SUBJECT MATTER
Int.Cl. A45D19/02(2006.01)i, A45D34/04(2006.01)i, A61Q5/00(2006.01)i, A61Q5/06(2006.01)i, A61K8/02(2006.01)i, A61K8/891(2006.01)i, A61K8/898(2006.01)i
FI: A45D34/04515A, A61K8/898, A61K8/02, A61Q5/06, A45D34/04515, A61Q5/00, A61K8/891, A45D19/02B
According to International Patent Classification (IPC) or to both national classification and IPC

B. FIELDS SEARCHED
Minimum documentation searched (classification system followed by classification symbols)
Int.Cl. A45D19/02, A45D34/04, A61Q5/00, A61Q5/06, A61K8/02, A61K8/891, A61K8/898

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y | JP 2008-63251 A (TOKIWA CORP.) 21.03.2008 (2008-03-21), claim 4, paragraphs [0010]-[0014], [0037]-[0039] | 1-7 |
| Y | WO 2006/006378 A1 (MITSUBISHI PENCIL CO., LTD.) 19.01.2006 (2006-01-19), abstract | 1-7 |
| Y | Microfilm of the specification and drawings annexed to the request of Japanese Utility Model Application No. 10088/1983 (Laid-open No. 117403/1984) (MURAKAMI, Tomo) 08.08.1984 (1984-08-08), specification, page 2, line 2 to page 3, line 2, fig. 1-3 | 1-7 |
| Y | JP 2014-529410 A (F.S.K. COLOR) 13.11.2014 (2014-11-13), paragraph [0023], fig. 1-3 | 3-7 |
| Y | JP 2019-6690 A (KAO CORPORATION) 17.01.2019 (2019-01-17), claim 1, paragraphs [0025], [0044] | 5-7 |

☐ Further documents are listed in the continuation of Box C.

☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 03.06.2020 | 16.06.2020 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**
Information on patent family members

International application No.
PCT/JP2020/015486

| | | |
|---|---|---|
| JP 2008-63251 A | 21.03.2008 | (Family: none) |
| WO 2006/006378 A1 | 19.01.2006 | US 2008/0083415 A1<br>abstract<br>EP 1769697 A1<br>KR 10-2007-0029797 A<br>CN 1980583 A<br>KR 10-1171129 B1 |
| JP 59-117403 U1 | 08.08.1984 | (Family: none) |
| JP 2014-529410 A | 13.11.2014 | US 2014/0096787 A1<br>paragraph [0029], fig. 1-3<br>WO 2012/169707 A1<br>EP 2719301 A1<br>KR 20-2012-0008734 U |
| JP 2019-6690 A | 17.01.2019 | (Family: none) |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2008063251 A
- JP 2012232104 A
- JP 2018140018 A